# EUROPÄISCHE PATENTSCHRIFT

(11) **EP 2 079 691 B1**
(45) Veröffentlichungstag und Bekanntmachung des Hinweises auf die Patenterteilung: **14.08.2013**
(21) Anmeldenummer: 07818703.6
(22) Anmeldetag: 04.10.2007
(51) Int. Cl.: C07D 209/94, A61K 31/403, A61P 35/00, A61P 29/00

(54) **SUBSTITUIERTE INDENO[1,2-B]INDOLDERIVATE ALS NEUE HEMMSTOFFE DER PROTEIN KINASE CK2 UND IHRE VERWENDUNG ALS TUMORTHERAPEUTIKA, CYTOSTATIKA UND DIAGNOSTIKA**
SUBSTITUTED INDENO[1,2-B]INDOLE DERIVATIVES AS NOVEL INHIBITORS OF PROTEIN KINASE CK2 AND THEIR USE AS TUMOUR THERAPEUTIC AGENTS, CYTOSTATICS AND DIAGNOSTIC AIDS
DERIVES D'INDENO[1,2-B]INDOLE SUBSTITUES COMME NOUVEAUX INHIBITEURS DE LA PROTEINE KINASE CK2 ET LEUR UTILISATION COMME AGENTS ANTITUMORAUX, CYTOSTATIQUES ET DIAGNOSTIQUES

(30) Priorität: 04.10.2006 DE 102006047231
(43) Veröffentlichungstag der Anmeldung: 22.07.2009
(73) Patentinhaber: Heinrich-Heine-Universität Düsseldorf, 40225 Düsseldorf (DE)
(72) Erfinder: HEMMERLING, Hans-Jörg, 12307 Berlin (DE); GÖTZ, Claudia, 66125 Saarbrücken (DE); JOSE, Joachim, 40589 Düsseldorf (DE)
(74) Vertreter: Weiss, Wolfgang
(86) Internationale Anmeldenummer: PCT/EP2007/008624
(87) Internationale Veröffentlichungsnummer: WO 2008/040547

(56) Entgegenhaltungen:
- EP-A- 1 266 887
- WO-A-90/15799
- SARNO ET AL: "Features and potentials of ATP-site directed CK2 inhibitors" BIOCHIMICA ET BIOPHYSICA ACTA (BBA) - PROTEINS & PROTEOMICS, ELSEVIER, Bd. 1754, Nr. 1-2, 30. Dezember 2005 (2005-12-30), Seiten 263-270, XP005214215 ISSN: 1570-9639 in der Anmeldung erwähnt
- SARNO STEFANIA ET AL: "Biochemical and three-dimensional-structural study of the specific inhibition of protein kinase CK2 by (5-oxo-5,6-dihydroindolo-(1,2-a)q uinazolin-7-yl)acetic acid (IQA)." BIOCHEMICAL JOURNAL, Bd. 374, Nr. 3, 15. September 2003 (2003-09-15), Seiten 639-646, XP002470109 ISSN: 0264-6021 in der Anmeldung erwähnt

## Beschreibung

Die vorliegende Erfindung betrifft Verbindungen der Struktur I, IA und II, ein Verfahren zu deren Synthese und pharmazeutische Zusammensetzungen, welche eine oder mehrere dieser Verbindungen enthalten. Ferner betrifft die Erfindung die Verwendung dieser Verbindungen zur Herstellung eines Medikamentes zur Behandlung von Tumoren und/oder Krebs und entzündlichen Erkrankung.

Nach Herz-Kreisauferkrankungen stellen bösartige Tumorerkrankungen die zweithäufigste Todesursache beim Menschen dar. Dabei sind Männer etwas häufiger betroffen als Frauen. Die Neuerkrankungsrate liegt in der Größenordung von 350 (Männer) und 250 (Frauen) pro 100.000 Einwohner pro Jahr bezogen auf die Weltbevölkerung, während die Mortalitätsrate bei etwa 170 (Männer) und 100 (Frauen) bezogen auf 100.000 Einwohner weltweit liegt. Die sich daraus ergebende immense Zahl von Betroffenen verdeutlicht die Notwendigkeit zur Entwicklung wirksamer Therapeutika neoplastischer Erkrankungen. Die neoplastischen Erkrankungen zeichnen sich durch eine stark erhöhte Zellproliferation aus und die Hemmung dieser Zellproliferation durch einen in einem Medikament enthaltenen Wirkstoff ist ein weltweit anerkannter Ansatzpunkt zur Tumortherapie. Solche in einem Medikament enthaltene Wirkstoffe sind dadurch gekennzeichnet, dass sie die Überlebensfähigkeit des Patienten verlängern, die mit dem Neoplasma verbundene erhöhte Zellproliferation hemmen oder einen Rückgang des Neoplasmas bewirken. Die Forschung auf diesem Gebiet zielt in erster Linie auf die Entwicklung von Medikamenten und Wirkstoffen, die beim Menschen therapeutisch einsetzbar sind. Oft werden dabei Verbindungen bei kleinen Säugern, z.B. Mäusen auf anti-neoplastische Wirksamkeit untersucht, wobei versucht wird, daraus Wirksamkeit gegen spezielle neoplastische Erkrankungen nicht nur bei diesen oder nahe verwandten Tieren, sondern auch beim Menschen vorher zu sagen.

Die Proteinkinase CK2 gehört zusammen mit der CK1, der Glycogensynthase-Kinase und den Cyclin-abhängigen Kinasen zu einer Gruppe second-messenger-unabhängiger Kinasen (1). Sie vermag Ser/Thr-Reste in einer vorwiegend sauren Umgebung zu phosphorylieren. Die CK2 ist eine hochkonservierte Kinase, die man von der Hefe bis zum Menschen hin findet. Ihre Aufgaben als ATP/GTP- Phosphotransferase nimmt sie in der Regel als Heterotetramer bestehend aus zwei katalytischen α- oder α'-Untereinheiten und zwei regulatorischen α-Untereinheiten wahr (2). Neben dieser heterotetrameren Form liegen die einzelnen Untereinheiten in der Zelle aber auch in freier Form oder an andere zelluläre Moleküle gebunden vor. In letzter Zeit mehren sich die Hinweise, dass auch in dieser Form die CK2 maßgebliche regulatorische Funktionen wahrnehmen kann (3). Sowohl als Holoenzym als auch als separate Untereinheiten beeinflusst sie durch die Bindung an Nukleinsäuren möglicherweise die Transkription, durch die Bindung an andere proliferationsrelevante Moleküle (p53, p21^{WAF1}, PP2A, mos, raf, Topoisomerase II) deren Aktivitäten und damit auch das Wachstum von Zellen (4). Während diese Funktion unabhängig von der Fähigkeit ist, Phosphatgruppen zu übertragen, bleibt die prominenteste Aufgabe der CK2 auf jeden Fall ihre enzymatische Aktivität. Bislang sind über 160 Substrate bekannt, die aus den verschiedensten Aufgabenbereichen des zellulären Geschehens stammen. Unter ihnen finden sich Moleküle, die für die Replikation, Transkription und Translation notwendig sind, Moleküle, die in Signaltransduktionsketten eingebunden sind, Proto-Onkogene (mdm2) und Wachstumssuppressoren (p53). Für eine Reihe dieser Substrate konnte nachgewiesen werden, dass ihre Aktivitäten nach der Phosphorylierung durch CK2 verändert sind. Aus ihren Substraten und dem Einfluß, den sie auf ihre Aktivitäten hat, läßt sich ableiten, daß die CK2 auch Einfluß auf das Wachstum von Zellen hat. Injiziert man z.B. Antikörper gegen die CK2 oder Antisense-Oligonukleotide in Zellen, so kommt es zu bestimmten Stadien des Zellzyklus zu einem Wachstumsarrest (5-7). Offensichtlich wird die Aktivität der CK2 dringend für die Übergänge G₀/G₁, G₁/S und für die Progression der S-Phase benötigt. Neuere Arbeiten belegen zudem ihre Notwendigkeit für die Progression durch die G2 und M-Phase. Durch Überexpression der katalytischen Untereinheit kommt es zu einer verstärkten Proliferation von Zellen, während es durch die Überexpression der regulatorischen Untereinheit zu einem schweren Wachstumsdefekt kommt, der mit einer Schrumpfung der Zelle einhergeht. Während der Embryonalentwicklung von Organismen wird eine gewebs- und zeitspezifische Expression und Aktivität der CK2 beobachtet, die auf eine Bedeutung bei der Differenzierung von Zellen schließen lässt. Neue Erkenntnisse deuten darauf hin, dass CK2 nicht nur an der Regulation der Proliferation, sondern auch an der Auslösung des programmierten Zelltodes (Apoptose) beteiligt ist (8).

Neben der Bedeutung der Protein Kinase CK2 für die Zellproliferation und das Überleben von Zellen, häufen sich in jüngster Zeit die Hinweise, dass das Enzym an der neoplastischen Transformation von Zellen und der Entstehung und Progression von Krebs beteiligt ist (9,10). In einer Vielzahl unterschiedlicher Tumoren lassen sich abnormal erhöhte Konzentrationen des Enzyms CK2 nachweisen. Dazu gehören unter anderem Prostatakrebs (11), Brustkrebs (12), Lungenkrebs (13) und andere Krebserkrankungen (14) des Menschen. Die direkte Beteiligung der Protein Kinase CK2 bei der malignen Transformation konnte auch in verschiedenen Tiermodellen verifiziert werden (15, 16). Die Ergebnisse dieser Untersuchungen und anderer deuten darauf hin, dass die Protein Kinase CK2 ein viel versprechender Ansatzpunkt für anti-neoplastische Wirkstoffe ist (17).

Trotz der eindeutigen Hinweise auf die Beteiligung der CK2 an der malignen Transformation und der Tumorentstehung und - progression sind derzeit nur wenige hoch aktive Inhibitoren des Enzyms verfügbar (18, 19). Dazu gehören Emodin (20), 4,5,6,7-tetrabromo-1-H-benzotriazole (TBB) (21), 2-Dimethylamino-4,5,6,7-tetrabrom-1H-benzimidazol (DMAT) (22), (5-Oxo-5,6-dihydro-indolo[1,2-a]chinoxalin-7-yl)essigsäure (IQA) (23) und Ellagsäure (24). Neben der moderaten Wirksamkeit (IC₅₀-Wert für Emodin: 2.0 µM, TBB: 0.9 µM) zeichnen sich diese dadurch aus, dass Ihre Wirksamkeit vorwiegend am Enzym der Ratte und nicht dem des Menschen untersucht wurde und dass sie aufgrund ihrer physiko-chemischen Eigenschaften nicht als Wirkstoff in einem Medikament, insbesondere zur peroralen Applikation in Frage kommen.

Es wurde gezeigt, dass CK2 ein Ziel für chronische entzündliche Erkrankungen, insbesondere Glomerulonephritis (26) und Lupus erythematodes (27), sein kann.

Indeno[1,2-b]indole sind mehrfach beansprucht: als Antioxidantien und Radikalscavenger (Sainsbury et al., EP-404536-A1), als Östrogenantagonisten (Miller et al. US Patent 6,107,292), als Kaliumkanalöffner (Antane et al. US Pat.Appl. 2001/0047026 A1), als Hemmstoffe der Topoisomerase II (Wierzbicki et al. US Patent 6,627,650), 4b,5,9b,10-Tetrahydro-indeno[1,2-b]indole als Antioxidantien (Sainsbury et al. WO 90/15799 und EP-409410-B1).

Ziel der vorliegenden Erfindung war es also, potente Inhibitoren zu synthetisieren, die aufgrund ihrer Struktur als Wirkstoffe zum Einsatz in Medikamenten zur Behandlung neoplastischer Erkrankungen in Frage kommen und deren Aktivität durch starke Hemmung der humanen Protein Kinase CK2 nachzuweisen. Ferner war es Ziel der vorliegenden Erfindung, Inhibitoren von CK2 bereitzustellen, welche zur Behandlung von chronischen entzündlichen Erkrankungen eingesetzt werden können.

Die erfindungsgemäßen Verbindungen zeichnen sich dadurch aus, dass sie neu sind, d.h. noch nicht beschrieben und beansprucht wurden und dass sie eine starke inhibierende Wirkung auf die humane Proteinkinase CK2 und andere Kinasen zeigen.

Die vorliegende Erfindung beschreibt die Synthese neuer Indeno[1,2-b]indolderivate mit der allgemeinen Struktur I, IA und II. Sie besitzen Hemmwirkung auf Proteinkinasen, insbesondere vom Typ Serin/Threonin-Kinasen und in dieser speziellen Ausführungsform auf die humane Proteinkinase CK2.

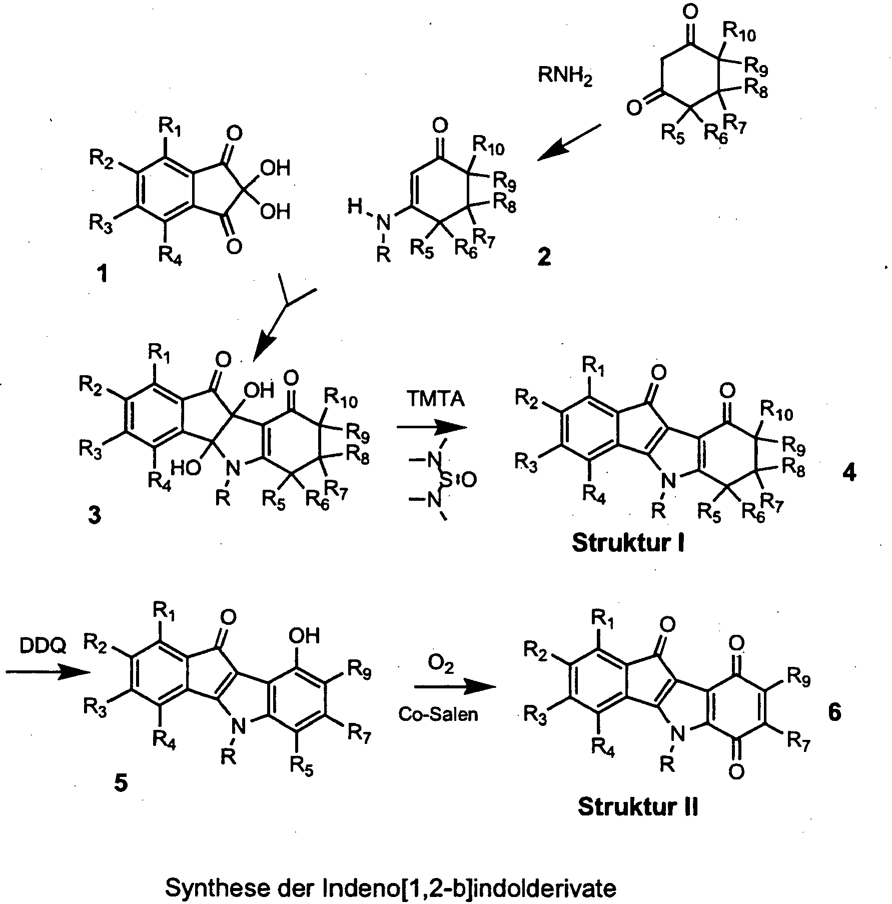

Ein erfindungsgemäßer Gegenstand ist ein neues, sehr effizientes Syntheseprinzip der Verbindungen mit der Struktur I, IA oder II, ausgehend von bekannten und leicht zugänglichen Edukten mit sehr guten bis guten Ausbeuten

### 1. Stufe:

Herstellung von cyclischen Enaminonen aus cyclische 1,3-Diketonen, durch Reaktion mit Ammoniak oder prim. Aminen und der Indantrione nach literaturbekannten Methoden:
R und R₁ - R₁₀ wie hierin beschrieben

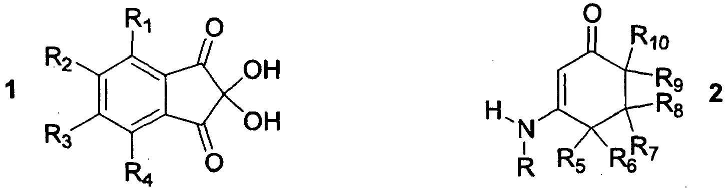

### 2. Stufe:

Reaktion der Enaminone 2 mit Indantrionhydrat 1 zu Additionsverbindungen 3

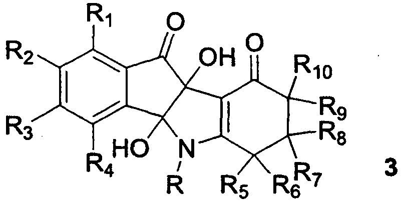

### 3. Stufe:

Deoxygenierung der Verbindungen 3 mit einem von uns entwickelten Reagenstyp: N,N,N',N'-Tetraalkylschwefligsäurediamide (TMTA: Alkyl = CH₃, C₂H₅)

Die Deoxygenierung wird vorzugsweise gemäß (29) durchgeführt.

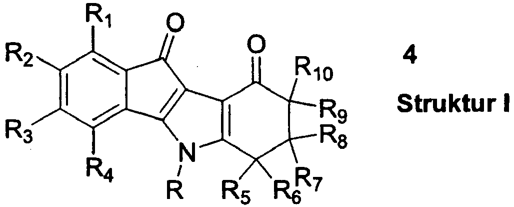

### 4. Stufe:

die Dehydrierung der Verbindungen **4** (Struktur I; R₆ = R₈ = R₁₀ = H) mit DDQ ergibt 5.

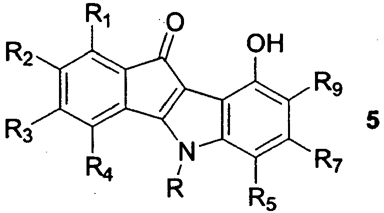

### 5. Stufe:

die katalytische Oxidation von Verbindungen **5** mit Sauerstoff (R₅ = H) führt zu **6** (Struktur II).

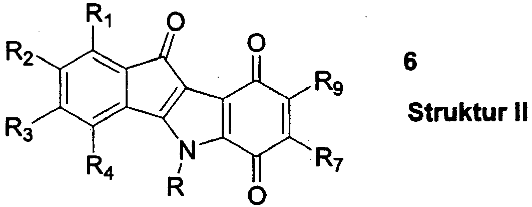

In einer besonders bevorzugten Ausführungsform betrifft die Erfindung die Verwendung der in der Erfindung beschriebenen neuen Verbindungen als Wirkstoffe in Medikamenten und/oder Arzneimitteln zur Behandlung insbesondere neoplastischer Erkrankungen.

Eine weitere besonders bevorzugten Ausführungsform betrifft die Verwendung der Verbindungen zur spezifischen Hemmung der humanen Proteinkinase CK2.

In einer weiteren Ausführungsform geht es um die in-vitro Hemmung von Proteinkinasen allgemein, im Speziellen um die Hemmung von Serin/Threoninkinasen durch die erfindungsgemäßen Verbindungen.

In einer weiteren besonderen Ausführungsform betrifft die Erfindung die Verwendung der beschriebenen neuen Verbindungen als Wirkstoffe in Medikamenten und/oder Arzneimitteln zur Behandlung weiterer Erkrankungen, insbesondere chronisch entzündlicher Erkrankungen wie zum Beispiel Glomerulonephritis oder/und Lupus erythematodes, degenerativer Erkrankungen oder anderer Erkrankungen, bei denen die Disbalance der Regulation der Zellproliferation ursächlich beteiligt ist.

Eine weitere erfindungsgemäße Verwendung der beschriebenen neuen Verbindungen betrifft deren Einsatz in Diagnostika zur Untersuchung der Rolle der Protein Kinase CK2 bei zellulären Prozessen, bei der Pathogenese von Erkrankungen oder bei der Ontogenese und sonstigen entwicklungsbiologischen Phänomenen und Zusammenhängen, sowie die Untersuchung der Rolle von Proteinkinasen allgemein und Serin/Threoninkinasen im Speziellen in den erwähnten Zusammenhängen. Die vorliegende Erfindung stellt Wirkstoffe zur Verwendung in Medikamenten und/oder Arzneimitteln zur Behandlung eines insbesondere an einem neoplastischen Erkrankungszustand leidenden Patienten bereit, die auch in Kombination mit anderen bereits bekannten insbesondere antineoplastisch wirksamen Verbindungen oder anderen Wirkstoffen eingesetzt werden können. Dadurch kann eine synergistische Wirkung ggfls. sogar eine über die Erwartung hinausgehende synergistische Wirkung erzielt werden.

Wie er hier verwendet wird, betrifft der Begriff "Patient" einen Warmblüter, wie einen Säuger, der von einem neoplastischen Erkrankungszustand befallen ist. Es ist selbstverständlich, Rinder, Pferde, Ratten, Mäuse, Katze, Hunde, Schafe, Ziegen und Menschen Tiere im Bedeutungsbereich des Begriffs sind.

Der Begriff "neoplastischer Erkrankungszustand", wie er hier verwendet wird, betrifft einen anormalen Zustand oder Beschwerden, die durch eine erhöhte Zellproliferation, Zellwachstum oder Neoplasmen gekennzeichnet sind. Neoplastische Erkrankungszustände, für die eine Therapie mit Medikamenten und/oder Arzneimitteln, in denen die erfindungsgemäßen Verbindungen enthalten sind im Speziellen anwendbar sein wird, umfassen: Leukämien, wie akute Lymphoblasten-, chronische Lymphozyten-, akute Myelomblasten- und chronische Myelozytenleukämie, aber sind nicht darauf beschränkt; Karzinome wie die der Brustdrüse, der Prostata, der Lunge, des Gebärmutterhalses, der Speiseröhre, des Magens, des Dünndarms, des Gehirns, des Dickdarms, aber sind nicht darauf beschränkt; Sarkome, wie Osteome, Osteosarkome, Lipome, Liposarkome, Hämagiome, und Hämangiosarkome, aber sind nicht darauf beschränkt; Melanome, einschließlich amelotischer und melanotischer; und gemischte Arten von Neoplasien, wie Karzinosarkome, Neoplasiearten des lymphatischen Gewebes, Follikelretikulumneoplasien, Zellsarkome und die Hodgkinsche Krankheit, aber sind nicht darauf beschränkt. Natürlich wird der Fachmann erkennen, das nicht jede der erfindungsgemäßen Verbindungen allein oder in Kombination mit anderen antineoplastisch wirkenden Verbindungen zur Behandlung mit jedem der neoplastischen Erkrankungszustände geeignet ist und kombiniert werden kann und dabei gleichermaßen wirksam sein wird. Die Auswahl der am besten geeigneten Verbindungen und der am besten geeigneten Kombinationen liegt im Rahmen der Fähigkeit eines Durchschnittfachmanns und wird von einer Vielzahl von Faktoren abhängen, die beispielsweise die Beurteilung von Ergebnissen, die bei gängigen Tierkrebsmodellen erhalten wurden, aber auch bei der Untersuchung der Wirkung einzelner Verbindungen zur Behandlung besonderer neoplastischer Erkrankungen einschließen.

Es wird erwartet, dass eine wirksame antineoplastische Menge einer der erfindungsgemäßen Verbindungen im Bereich von etwa 1 Milligramm pro Kilogramm pro Tag (mg/kg/Tag) bis etwa 100 mg/kg/Tag schwankt und vorzugsweise etwa 0.1 bis etwa 20 mg/kg/Tag entspricht.

Wie er hier verwendet wird, betrifft der Begriff "wirksame antineoplastische Menge" eine Menge, die nach Verabreichung einer Einzel- oder Mehrfachdosis an den Patienten zum Bekämpfen des Wachstums des Neoplasmas oder zum Verlängern der Überlebensfähigkeit des Patienten über die, die in Abwesenheiten einer solchen Behandlung erwartet wird, hinaus wirksam ist. Wie hier verwendet, bezieht sich "Bekämpfen des Wachstums" des Neoplasmas auf ein Verlangsamen, Unterbrechen, Hemmen oder Anhalten seines Wachstums und bedeutet nicht notwendigerweise eine völlige Eliminierung des Neoplasmas.

Eine Einzel- oder Mehrfachdosis kann mit einer der erfindungsgemäßen Verbindungen allein oder in Kombination mit anderen insbesondere antineoplatisch wirkenden Verbindungen verabreicht werden.

Beim Ausführen der Behandlung eines Patienten, der von einem vorstehend beschriebenen Erkrankungszustand befallen ist, kann eine der erfindungsgemäßen Verbindungen in einer beliebigen Form und Weise, die die Verbindung in wirksamen Mengen biologisch verfügbar macht, einschließlich oraler und parenteraler Wege verabreicht werden. Zum Beispiel kann sie oral, subkutan, intramuskulär, intravenös, transdermal, intranasal, rektal und dergleichen verabreicht werden. Die orale Verabreichung ist im Allgemeinen bevorzugt. Ein Fachmann im Herstellen von Formulierungen kann leicht die passende Form und Art der Verabreichung in Abhängigkeit von den besonderen Umständen, die den zu behandelnden Erkrankungszustand, das Stadium der Erkrankung, die Form der Verabreichung der Verbindung in Kombination mit anderen insbesondere antineoplastisch wirksamen Wirkstoffen oder allein, die ausgewählte Art der gemeinsamen Verabreichung und dergleichen einschließen, auswählen.

In einer bevorzugten Ausführungsform der vorliegenden Erfindung erfolgt die Verabreichung der erfindungsgemäßen Verbindungen oral in einer Formulierung folgender Zusammensetzung: Verbindung 10 g, Hydroxypropylcellulose 2 g, Getreidestärke 11 g, Lactose 100 g, Magnesiumstearat 3 g und Talkum 3 g, wobei die Bestandteile in den beschriebenen Mengen und Mengenverhältnissen ausreichen, um 1000 Tabletten mit 10 mg Wirkstoffgehalt herzustellen.

Die erfindungsgemäßen Verbindungen können, solange an sich wirksam, in Form ihrer pharmazeutisch verträglichen Säureadditionssalze zum Zweck der Stabilität, der Bequemlichkeit der Kristallisation, einer erhöhten Löslichkeit und dergleichen formuliert und verabreicht werden.

Eine weitere besondere Ausführungsform der Erfindung betrifft die Verwendung der erfindungsgemäßen Verbindungen als Edukte in weiteren Syntheseschritten zur Verbesserung der Löslichkeit, der Bioverfügbarkeit, der physiko-chemischen Eigenschaften und dergleichen.

Gegenstand der Erfindung sind auch die folgenden spezifischen Ausführungsformen:
Ausführungsform 1: Verbindungen der Struktur I und der Struktur II 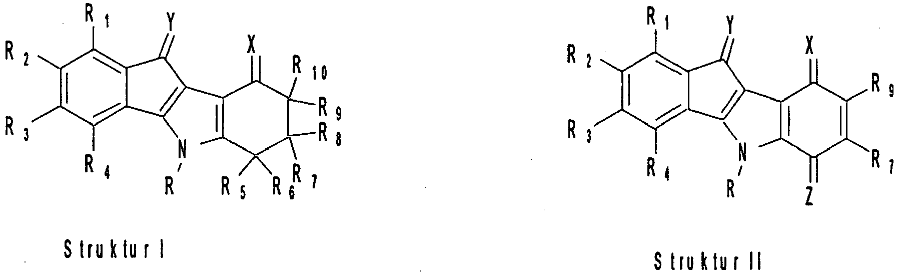 **in der R sein kann**
   ein **Wasserstoffatom**
   eine **Alkylgruppe,** (C1 - C8) geradkettig oder verzweigt,
   **Cycloalkylgruppe** (C1 - C8) oder
   **Alkenylgruppe** (C₁ - C₈) geradkettig oder verzweigt oder
   **Cycloalkenylgruppe** (C1 -C8) oder
   **Arylgruppe** oder
   **Neteroarylgruppe,**
   die gegebenenfalls durch Hydroxy-, Cyano-, Nitro-, Halogen-, Carboxy-, Sulfonsäure-, Carboxamido- und Thiocarboxamidogruppen, Sulfonamidgruppen (CONR₁₁R₁₂, CSNR₁₁R₁₂, SO₂NR₁₁R₁₂ (R₁₁, R₁₂ können gleich oder verschieden Wasserstoffatome, geradkettige oder verzweigte Alkylgruppen (C1 - C8) oder Cycloalkylgruppen (C2 - C8)), Aminogruppen NR₁₃R₁₄ (R₁₃, R₁₄ identisch oder verschieden können sein geradkettige oder verzweigte Alkylgruppen (C1 - C8 oder Cycloalkylgruppen (C2 - C8) oder gemeinsam mit dem sie tragenden Stickstoffatom eine Stickstoffheterocyclus bildend, substituiert sind, Alkoxycarbonylgruppe COOR₁₅ (R₁₅: geradkettige oder verzweigte Alkylgruppen (C1 - C8) oder Cycloalkygruppen (C1 - C8), gegebenenfalls substituiert durch Hydroxy-, Haloge-, Cyano-, Nitro-, Carboxy-, Carbamoyl- und Thiocarbamoylgruppen, Sulfamoylgruppen CO(NR₁₆R₁₇, CSNR₁₆R₁₇, SO₂NR₁₆R₁₇, (R₁₆, R₁₇ können gleich oder verschieden, Wasserstoffatome, geradkettige oder verzweigte Alkylgruppen (C1 - C8) oder Cycloalkylgruppen (C2 - C8) sein), Aminogruppen NR₁₈R₁₉ (R₁₈, R₁₉ gleich oder verschieden, können sein Wasserstoff, lineare oder verzweigte Alkylgruppen (C1 - C8 oder Cycloalkylgruppen (C2 - C8) oder gemeinsam mit dem sie tragenden Stickstoffatom eine Stickstoffheterocyclus bildend), eine Aminoalkyloxyalkyl NR₂₀R₂₁, (R₂₀, R₂₁ können gleich oder verschieden Wasserstoffatome,geradkettige oder verzweigte Alkylgruppen (C1- C8), oder gemeinsam mit dem sie tragenden Stickstoffatom einen Stickstoffheterocyclus bildend), substituiert sind,
   eine **Stickstofffunktion NR₂₂R₂₃**
   (R₂₂, R₂₃ können Wasserstoff oder gleiche oder verschiedene geradkettige oder verzweigte Alkylgruppen (C1 - C8) oder Cycloalkylgruppen (C2 - C8), Arylgruppen, Heteroarylgruppen), Alkoxygruppen OR₂₄, (R₂₄ kann sein eine geradkettige oder verzweigte Alkylgruppe), gegebenenfalls substituiert durch Hydroxy-, Halogen-, Cyano-, Nitro-, Carboxy-, Carboxamido- und Thiocarboxamidogruppen, Sulfonamidogruppen (CO(NR₂₅R₂₆, CSNR₂₅R₂₆, SO₂NR₂₅R₂₆, (mit R₂₅R₂₆, gleich oder verschieden können sein Wasserstoff, geradkettige oder verzweigte Alkylgrupen (C1- C8), Arylgruppen, Heteroarylgruppen oder gemeinsam mit dem sie tragenden Stickstoffatom einen Stickstoffheterocyclus bildend), Alkoxycarbonylgruppe (C1 - C8) COOR₂₇ (mit R₂₇ geradkettige oder verzweigte Alkylgruppen(C1-C8,Cycloalkylgruppen, Arylgruppen, Heteroarylgruppen, gegebenenfalls substituiert durch Hydroxy-, Halogen-, Cyano-, Nitro-, Carboxy-, Carboxamido- und Thiocarboxamidogruppen, Sulfonamidogruppen (CO(NR₂₈R₂₉, CSNR₂₈R₂₉, SO₂NR₂₈R₂₉, (mit R₂₈R₂₉ können gleich oder verschieden sein, Wasserstoff, geradkettige oder verzweigte Alkylgrupen(C1-C8), oder gemeinsam mit dem sie tragenden Stickstoffatom einen Stickstoffheterocyclus bildend), eine Aminoalkyloxyalkyl NR₃₀R₃₁, (R₃₀, R₃₁ können sein gleiche oder verschiedene geradkettige oder verzweigte Alkylgruppen (C1 - C8) oder gemeinsammit dem sie tragenden Stickstoffatom einen Stickstoffheterocyclus bilden oder Aryl- oder Heteroarylgruppen) sein,
   eine **Sauerstofffunktion OR₃₂**
   (R₃₂ kann Wasserstoff oder eine geradkettige oder verzweigte Alkylgruppe (C1 - C8) oder Cycloalkylgruppe (C2 - C8), gegebenenfalls substituiert durch Hydroxy-, Halogen-, Cyano-, Nitro-, Carboxy-, Carbamoyl- und Thiocarbamoylgruppen, Sulfamoylgruppen CO(NR₁₆R₁₇, CSNR₁₆R₁₇, SO₂NR₁₆R₁₇, Alkoxygruppen (R₂₉ kann sein eine lineare oder verzweigte Alkylgruppen (C1 - C8) oder Cycloalkylgruppen (C2 - C8), eine Alkoxycarbonylgruppe, lineare oder verzweigte (C1 - C8) oder Cycloalkyl (C1 - C8), eine Aminoalkyloxyalkyl NR₃₀R₃₁ (R₃₀, R₃₁ können sein identische oder verschiedene lineare oder verzweigte Alkylgruppen (C1-C8), oder mit dem sie tragenden Stickstoffatom einen Stickstoffheterocyclus bilden) oder Aryl- oder Heteroarylgruppen) sein.
   **R₁ - R₁₀** können gleichartig oder verschieden sein.
   Jedes kann sein
   ein **Wasserstoffatom,**
   eine **Alkylgruppe**(C1 - C8) geradkettig oder verzweigt oder
   eine **Cycloalkylgruppe** (C1 - C8) oder
   eine **Alkenylgruppe** (C₁ - C₈) geradkettig oder verzweigt oder
   eine **Cycloalkenylgruppe** (C1 -C8) oder
   eine **Arylgruppe,** oder
   eine **Heteroarylgruppe,**
   die gegenenfalls durch Hydroxy-, Cyano-, Nitro-, Halogen-, Carboxy-, Sulfonsäure-, Carboxamido- und Thiocarboxamidogruppen, Sulfonamidgruppen (CONR₃₂R₃₃, CSNR₃₂R₃₃, SO₂NR₃₂R₃₃ (R₃₂, R₃₃ können gleich oder verschieden Wasserstoffatome, geradkettige oder verzweigte Alkylgruppen (C1 - C8) oder Cycloalkylgruppen (C2 - C8)), Aminogruppen NR₃₄R₃₅ (R₃₄, R₃₅ identisch oder verschieden können sein geradkettige oder verzweigte Alkylgruppen (C1 - C8 oder Cycloalkylgruppen (C2 - C8) oder gemeinsam mit dem sie tragenden Stickstoffatom einen Stickstoffheterocyclus bildend, substituiert sind), Alkoxycarbonylgruppe COOR₃₆ (R₃₆: geradkettige oder verzweigte Alkylgruppen (C1 - C8) oder Cycloalkygruppen (C1 - C8), gegebenenfalls substituiert durch Hydroxy-, Haloge-, Cyano-, Nitro-, Carboxy-, Carbamoyl- und Thiocarbamoylgruppen, Sulfamoylgruppen CO(NR₃₇R₃₈, CSNR₃₇R₃₈, SO₂NR₃₇R₃₈, (R₃₇, R₃₈ können gleich oder verschieden, Wasserstoffatome, geradkettige oder verzweigte Alkylgruppen (C1 - C8) oder Cycloalkylgruppen (C2 - C8) sein), Aminogruppen NR₃₉R₄₀ (R₃₉, R₄₀ gleich oder verschieden, können sein Wasserstoff, lineare oder verzweigte Alkylgruppen (C1 - C8 oder Cycloalkylgruppen (C2 - C8) oder gemeinsam mit dem sie tragenden Stickstoffatom eine Stickstoffheterocyclus bildend), eine Aminoalkyloxyalkyl NR₄₁ R₄₂, (R₄₁, R₄₂ können gleich oder verschieden Wasserstoffatome, geradkettige oder verzweigte Alkylgrupen (C1- C8), oder gemeinsam mit dem sie tragenden Stickstoffatom einen Stickstoffheterocyclus bildend), sein gegebenenfalls substituiert durch eine Aryl-, Carboxy- oder durch eine Alkoxycarbonylgruppe, linear oder verzweigt (C1 - C8) substituiert sind.
   eine **Hydroxygruppe**
   eine **Acyloxycarbonylgruppe,** OCOR₄₃
   (R₄₃ kann eine geradkettige oder verzweigte Alkylgruppe (C1 - C8) oder Cycloalkylgruppe oder Arylgruppeoder oder Heteroarylgruppe sein).
   eine **Carboxygruppe**
   eine **Aminofunktion** NR₄₄R₄₅,
   in der R₄₄ R₄₅ gleich oder verschieden, geradkettige oder verzweigte Alkylgruppen (C1 - C8) oder Cycloalkyl (C1 - C8), Arylgruppen oder Heteroarylgruppen sein können, gegebenenfalls substituiert mit einer Aminogruppe NR₄₆R₄₇, in der R₄₆,R₄₇ gleich oder verschieden sein können und geradkettige oder verzweigte (C1 - C8) Alkylgruppen (C1 - C8) oder Cycloalkyl (C1 - C8), Arylgruppen oder Heteroarylgruppen, sein können.
   eine **Alkoxygruppe** mit geradkettige oder verzweigte Alkylgruppen (C1 - C8) oder Cycloalkyl (C1 - C8), Arylgruppen oder Heteroarylgruppen
   eine **Alkenyloxygruppe** mit geradkettige oder verzweigte Alkylgruppen (C1 - C8) oder Cycloalkyl (C1 - C8), Arylgruppen oder Heteroarylgruppen, gegebenenfalls substituiert durch eine Arylgruppe oder eine Aminofunktion NR₃₆R₃₇, in der R₃₆, R₃₇ identisch oder verschieden sein können, jede von ihnen lineare oder verzweigte (C1 - C8) Alkylgruppen oder Cycloalkylgruppen (C1 - C8) tragen könne oder zusammen mit dem Stickstoffatom einen stickstoffhaltigen Heterocyclus bilden
   oder einer der Substituenten R₁ - R₁₀ bildet mit einem benachbarten R₁ -R₁₀ eine **Alkylendioxygruppe**
   X kann sein
   **Sauerstoff**
   eine **Stickstofffunktion** NR₄₀,
   in der R₄₀ ein Wasserstoffatom, eine Alkylgruppe linear oder verzweigt (C1 -C8) oder eine Cycloalkylgruppe (C1 - C8) oder eine Aryl- oder eine Arylalkyl(Cl - C8)gruppe sein kann, in der die Alkylkette linear oder verzweigt sein kann. Optional kann die Alkylgruppe eine oder mehrere Mehrfachbindungen tragen und / oder substituiert sein mit mehreren Gruppen, die identisch oder verschieden sein können und durch Aryl, Heteroaryl-, Cycloalkyl- (C3 - C8), Cyano-, Nitro- oder eine Aminofunktion NR₄₁R₄₂ ( R₄₁, R₄₂ können sein identische oder verschiedene Alkylgruppen (C1 - C8), linear oder verzweigt oder Cycloalkyl (C1 - C8) oder bilden mit dem sie tragende Stickstoffatom einen Heterocyclus) zusammen oder einzeln substiuiert sein kann. Alkylgruppen oder Cycloalkylgruppen (C1 - C8) sein können oder mit dem sie tragenden Stickstoffatom einen stickstoffhaltigen Heterocyclus bilden, gegebenenfalls substituiert durch Hydroxy-, Halogen-, Cyano-, Nitro-, Carboxy-, Sulfonyl-, Carboxamido- und Thiocarboxamidogruppen, Sulfonamidgruppen CONR₃₇R₃₈, CSNR₃₇R₃₈, SO₂NR₃₇R₃₈) sein kann.
   **Y kann sein**
   **Sauerstoff,**
   eine **Stickstofffunktion** NR₄₈,
   in der R₄₈ ein Wasserstoffatom, eine Alkylgruppe linear oder verzweigt (C1 -C8), oder eine Aryl- oder eine Arylalkyl(C1 - C8)gruppe repräsentiert, in der die Alkylkette linear oder verzweigt sein kann. Optional kann die Alkylgruppe eine oder mehrere Mehrfachbindungen tragen und / oder substituiert sein mit mehreren Gruppen, die identisch oder verschieden sein können und Aryl, Heteroaryl-, Cycloalkyl- (C3 - C8), Cyano- oder eine Aminofunktion NR₄₉R₅₀ (R₄₉, R₅₀ können sein identische oder verschiedene Alkyl- (C1 - C8) oder Cycloalkylgruppen (C2 - C8, linear oder verzweigt oder bilden mit dem sie tragende Stickstoffatom einen Heterocyclus, sein kann.
   **Z kann sein**
   **Sauerstoff**
   eine **Stickstofffunktion** NR₄₉,
   in dem R₄₉ ein Wasserstoffatom, eine Alkylgruppe geradkettig oder verzweigt (C1 -C8), oder eine Aryl- oder eine Arylalkyl(C1 - C8)gruppe repräsentiert, in dem die Alkylkette geradkettig oder verzweigt sein kann. Optional kann die Alkylgruppe eine oder mehrere Mehrfachbindungen tragen
   und / oder substituiert sein mit mehreren Gruppen, die identisch oder verschieden sein können und Aryl, Heteroaryl-, Cycloalkyl- (C3 - C8), Cyano-, Nitro-; oder eine Aminofunktion NR₄₉R₅₀ (R₄₉, R₅₀ können sein identische oder verschiedene Alkyl- (C1 - C8) oder Cycloalkylgruppen (C2 - C8, linear oder verzweigt oder bilden mit dem sie tragende Stickstoffatom einen Heterocyclus.
   Unter **Isomeren** sind optische Isomere und geometrische Isomere an Doppelbindungen zu verstehen. Die Ausführungsform schließt sowohl Isomerengemische als auch die reinen Isomeren (Diastereomere, Enantiomere, E/Z-Isomere) ein.
   Unter **Arylgruppen** sind Phenyl, Naphthylgruppen zu verstehen, die mit einem oder meheren identisch oder verschiedenen Substituenten, wie Halogen, Alkoxy-, Phenoxy-, Nitro-, Cyano-, Aminogruppen NR₄₃R₄₄ (R₄₃R₄₄ können sein identische oder verschiedene Alkylgruppen (C1 - C8), linear oder verzweigt oder Cycloalkyl (C1 - C8) oder bilden mit dem sie tragende Stickstoffatom einen Heterocyclus) oder substituiert mit Arylalkyl- und/oder Alkylgruppen, linear oder verzweigt (C1 - C8) oder Cycloalkyl (C1 - C8) oder Alkylendioxy (C1 - C2), Aminoalk(C1 - C8)oxygruppen NR₄₅R₄₆ (R₄₅,R₄₆ können sein identische oder verschiedene Alkylgruppen (C1 - C8), linear oder verzweigt oder Cycloalkyl (C1 - C8) oder bilden mit dem sie tragende Stickstoffatom einen Heterocyclus) zusammen oder einzeln substiuiert sein können.
   Unter Heteroarylgruppen sind aromatische mono- oder bicyclische Ringsystem mit 5 bis 12 Ringgliedern, die 1 - 3 Heteroatome (O, N, S) enthalten zu verstehen, die mit einem oder meheren identisch oder verschiedenen Substituenten, wie Halogen, Alkoxy-, Phenoxy-, Nitro-, Cyano-, Aminogruppen NR₄₃R₄₄ (R₄₃R₄₄ können sein identische oder verschiedene Alkylgruppen (C1 - C8), linear oder verzweigt oder Cycloalkyl (C1 - C8) oder bilden mit dem sie tragende Stickstoffatom einen Heterocyclus) oder substituiert mit Arylalkyl- und/oder Alkylgruppen, linear oder verzweigt (C1 - C8) oder Cycloalkyl (C1 - C8) oder Alkylendioxy (C1 - C2), Aminoalk(C1 - C8)oxygruppen NR₄₅R₄₆ (R₄₅,R₄₆ können sein identische oder verschiedene Alkylgruppen (C1 - C8), linear oder verzweigt oder Cycloalkyl (C1 - C8) oder bilden mit dem sie tragende Stickstoffatom einen Heterocyclus) zusammen oder einzeln substiuiert sein können..
   Unter einem stickstoffhaltigen Heterocyclus ist ein gesättigter Monocyclus mit 5 - 7 Ringglieder zu verstehen und der 1-3 Heteroatome (O,N, S) enthält.
Ausführungsform 2: In einer besonders bevorzugten Ausführungsform der Erfindung Verbindungen nach Ausführungsform 1, gekennzeichnet dadurch, dass X ein Sauerstoffatom ist.
Ausführungsform 3: In einer weiteren besonders bevorzugten Ausführungsform der Erfindung Verbindungen nach Ausführungsform 1, gekennzeichnet dadurch dass Y ein Sauerstoffatom ist.
Ausführungsform 4: In einer weiteren besonders bevorzugten Ausführungsform der Erfindung Verbindungen nach Ausführungsform 1, gekennzeichnet dadurch dass Z ein Sauerstoffatom ist.
Ausführungsform 5: In einer weiteren besonders bevorzugten Ausführungsform der Erfindung Verbindungen nach Ausführungsform 1, gekennzeichnet dadurch dass X = Y = Sauerstoff ist.
Ausführungsform 6: In einer weiteren besonders bevorzugten Ausführungsform der Erfindung Verbindungen nach Ausführungsform, gekennzeichnet dadurch dass R₅ - R₁₀ Wasserstoff ist.
Ausführungsform 7: In einer weiteren besonders bevorzugten Ausführungsform der Erfindung Verbindungen nach Ausführungsform 1, gekennzeichnet dadurch dass X = Y = Z = Sauerstoff ist.
Ausführungsform 8: In einer weiteren besonders bevorzugten Ausführungsform der Erfindung Verbindungen nach Ausführungsform 1, gekennzeichnet dadurch dass R₇ = R₉ = Wasserstoff ist.
Ausführungsform 9: In einer weiteren besonders bevorzugten Ausführungsform der Erfindung Verbindungen nach Ausführungsform 1, gekennzeichnet dadurch dass R₁ - R₁₀ identisch oder verschieden sein können und Wasserstoff, Alkyl- oder Alkoxygruppen sind.
Ausführungsform 10: In einer weiteren besonders bevorzugten Ausführungsform der Erfindung Verbindungen nach Ausführungsform 1, gekennzeichnet dadurch dass R ein Wasserstoffatom, eine Alkylgruppe, linear oder verzweigt (C1 - C8), eine Arylgruppe oder eine Arylalkylgruppe, linear oder verzweigt (C1 - C8) ist.
Ausführungsform 11: In einer weiteren besonders bevorzugten Ausführungsform der Erfindung die Verbindung nach Ausführungsform 1 ( Struktur I) mit der Bezeichnung (IUPAC): 5-*Isopropyl-5,6,7,8-tetrahydro-indeno[1,2-b]indol-9,10-dion* oder ein Salz dieser Verbindung mit einer pharmazeutisch gebräuchlichen Säure.
Ausführungsform 12: In einer weiteren besonders bevorzugten Ausführungsform der Erfindung die Verbindung nach Ausführungsform 1 (Struktur I) mit der Bezeichnung (IUPAC): 5-*Phenethy*/*-5,6,7,8-tetrahydro-indenol[1,2-b]indol-9,10-dion* oder ein Salz dieser Verbindung mit einer pharmazeutisch gebräuchlichen Säure.
Ausführungsform 13: In einer weiteren besonders bevorzugten Ausführungsform der Erfindung die Verbindung nach Ausführungsform 1 (Struktur I) mit der Bezeichnung (IUPAC): *5-(1-Phenyl-ethyl)-5,6,7,8-tetrahydro-indeno[1,2-b]indol-9,10-dion,* ihrer Isomeren oder ein Salz dieser Verbindung mit einer pharmazeutisch gebräuchlichen Säure.
Ausführungsform 14: In einer weiteren besonders bevorzugten Ausführungsform der Erfindung die Verbindung nach Ausführungsform 1 (Struktur I) mit der Bezeichnung (IUPAC): 5-*Phenyl.5,6,7,8-tetrahydro-indeno[1,2-b]indol-9,10-dion,* oder ein Salz dieser Verbindung mit einer pharmazeutisch gebräuchlichen Säure.
Ausführungsform 15: In einer weiteren besonders bevorzugten Ausführungsform der Erfindung die Verbindung nach Ausführungsform 1 (Struktur II) mit der Bezeichnung (IUPAC): 5-*Isopropyl-5H-indeno[1,2-b]indol-6,9,10-trion* oder ein Salz dieser Verbindung mit einer pharmazeutisch gebräuchlichen Säure.
Ausführungsform 16: In einer weiteren besonders bevorzugten Ausführungsform der Erfindung die Verbindung nach Ausführungsform 1 (Struktur II) mit der Bezeichnung (IUPAC): 5-*Benzyl-5,6,9,10-tetrahydroindeno[1,2-b]indol-6,9,10-trion* oder ein Salz dieser Verbindung mit einer pharmazeutisch gebräuchlichen Säure.
Ausführungsform 17: In einer weiteren besonders bevorzugten Ausführungsform der Erfindung die Verbindung nach Ausführungsform 1 (Struktur II) mit der Bezeichnung (IUPAC): 5-*Phenethyl-5H-indeno[1,2-b]indol-6,9,10-trion* oder ein Salz dieser Verbindung mit einer pharmazeutisch gebräuchlichen Säure.
Ausführungsform 18: Verbindungen gemäß Ausführungsform 1, gekennzeichnet dadurch dass sie sich aus der enolisierten Form der Struktur I (mit XR₁₁ = OR¹¹ im Folgenden mit Struktur IA bezeichnet) ergeben und dass R¹¹ den in Ausführungsform 1 zitierten Gruppen entspricht.
Ausführungsform 23: Verwendung der Verbindungen nach Ausführungsform 1- 18 zum Einsatz in Diagnostika zur Untersuchung der Rolle der Protein Kinase CK2 bei zellulären Prozessen, bei der Pathogenese von Erkrankungen oder bei der Ontogenese und sonstigen entwicklungsbiologischen Phänomenen und Zusammenhängen, sowie zur Untersuchung der Rolle von Proteinkinasen allgemein und Serin/Threoninkinasen im Speziellen in den erwähnten Zusammenhängen.
Ausführungsform 26: Eine pharmazeutische Zubereitung, dadurch gekennzeichnet dass sie als Wirkstoff Verbindungen der Struktur I, Struktur IA oder Struktur II nach Ausführungsform 1 -18 allein oder in Kombination mit anderen Wirkstoffen in einer wirksamen Dosierung enthält und mit pharmazeutisch gebräuchlichen Zusätzen und Hilfsstoffen bereitet wurde.
Die folgenden repräsentativen Beispiele und Tabellen demonstrieren die Erfindung. Beispiele sollen nur veranschaulichend sein und sollen den Bereich der Erfindung in keiner Weise beschränken.

### Legenden

**Tabelle 1** ist eine Zusammenstellung von erfindungsgemäßen Verbindungen und Vergleichsverbindungen.

**Tabelle 2:** IC₅₀-Werte ausgesuchter Indeno[1,2,-b]indole für die humane Proteinkinase CK2 (Holoenzym).

**Tabelle 3:** Proliferation von Tumorzelllinien nach Inkubation mit verschiedenen Indeno[1,2-b]indolen.

**Tabelle 4:** IC₅₀-Werte der Zellproliferation ausgesuchter Indeno[1,2,-b]indole für verschiedene Tumorzelllinien.

**Tabelle 5:** IC₅₀-Werte [µM] ausgesuchter Indeno[1,2,-b]indole von verschiedenen humanen Proteinkinasen.

### Beispiel 1: 5-Isopropyl-5,6,7,8-tetrahydro-indeno[1,2-b]indol-9,10-dion 4b

Die Strukturen der Verbindungen wurden in diesem und in den Beispielen 2 bis 26 durch spektrale Daten (NMR, IR, MS) und Mikroanalysen, in Einzelfällen auch durch Röntgenstruktur belegt.

### 1. Stufe: 3-Isopropylamino-cyclohex-2-enon nach

Äquimolare Mengen Isopropylamin und Cyclohexan-1,3-dion wurden in Benzol gelöst und unter Zusatz von p-Toluolsulfonsäure am Wasserabscheider zum Sieden erhitzt. Nach Beendigung der Wasserabscheidung wurde der Ansatz gründlich mit Wasser gewaschen, das Lösungsmittel abgezogen und der Rückstand umkristallisiert.

### 2. Stufe: 4b,9b-Dihydroxy-5-isopropyl-4b,5,6,7,8,9b-hexahydro-indeno[1,2-b]indol-9,10-dion

Äquimolare Mengen 3-Isopropylamino-cyclohex-2-enon und Indantrionhydrat (Ninhydrin) wurden in Chloroform gelöst und 12 Std. bei Raumtemperatur gerührt. Dann wurde das Lösungsmittel abgezogen und der ölige Rückstand aus Aceton zur Kristallisation gebracht.

### 3. Stufe: 5-Isopropyl-5,6,7,8-tetrahydro-indeno[1,2-b]indol-9,10-dion 4b

Die nach Stufe 2 erhaltene Verbindung (0.01 Mol) wurden in 20 ml N,N-Dimethylformamid DMF gelöst. Nach Zugabe von 5 ml Essigsäure wurden 0.04 Mol N,N,N'N'-Tetramethylsulfoximid, gelöst in 5 ml DMF zügig zugegeben. Der Ansatz wird 3 Std. gerührt, dann in 500 ml Wasser gegeben und der sich abscheidende Niederschlag abfiltriert. Er wird getrocknet und umkristallisiert.
Schmp.: 201°C. (Ethylacetat).

| | |
|---|---|
| Mikroanalyse: C₁₈H₁₉NO₄ (313.34) | ber.*: C 68.99, H 6.11, N 4.47; |
| | gef.*: C 68.95, H 6.17, N 4.52. |

| | |
|---|---|
| * ber. = berechnet, gef. = gefunden | |

### 4. Stufe: 9-Hydroxy-5-isopropyl-5H-indeno[1,2-b]indol-10-on

Die nach Stufe 3 erhaltene Verbindung (1.0 g, 3.61 mMol) wurden in 150 ml Dioxan gelöst und DDQ (0.91g, 4 mMol), gelöst in Dioxan, dazugegeben. Der Ansatz wurde 4 Std. auf 70 °C. erwärmt. Nach Filtration wurde der Ansatz eingeengt und sc an SiO₂/Ethylacetat aufgereinigt.
Schmp.: 182 °C. (Methanol).

| | |
|---|---|
| Mikroanalyse: C₁₈H₁₅NO₂ (277.32) | ber.: C 77.96, H 5.45, N 5.05; |
| | gef.: C 78.18, H 5.66, N 5.00. |

### 5. Stufe: 5-Isopropyl-5H-indeno[1,2-b]indol-6,9,10-trion 6b

Die nach Stufe 4 erhaltene Verbindung (0.8g, 2.5 mMol) wurde in 100 ml DMF gelöst, 80 mg Co-Salen zugegeben und bei 70 °C. Sauerstoff eingeleitet. Nach 4 Std. war die Ausgangssubstanz verschwunden. Ansatz wurde eingeengt, in Chloroform gelöst und sc gereinigt. (SiO₂/Ethylacetat). Schmp.:

| | |
|---|---|
| Mikroanalyse: C₂₀H₂₂N₄O₂ (350.41) | ber.: C 74.22, H 4.50, N 4.81; |
| | gef.: C 74.03, H 4.39, N 4.74. |

### Beispiel 2: 5-Benzyl-5,6,7,8-tetrahydro-indeno[1,2-b]indol-9,10-dion

Die Verbindung wird in Übereinstimmung mit dem o. a. Verfahren erhalten. Schmp.: 185 °C. (Ethylacetat).

| | |
|---|---|
| Mikroanalyse C₂₂H₁₇NO₂ (327.38) | ber. : C 80.71, H 5.23, N 4.28; |
| | gef.: C 80.81, H 5.20, N 4.24. |

### Beispiel 3: 5-Benzyl-5,6,9,10-tetrahydroindeno[,2-b]indol-6,9,10-trion 6c Die Verbindung wird in Übereinstimmung mit dem o. a. Verfahren erhalten. Schmp.: 224 °C. (2-Propanol).

HRMS: ber.: 339.08954; gef.: 339.08955.

### Beispiel 4: 5,6,7,8-Tetrahydroindeno[9,2-b]indol-9,10-dion

Die Verbindung wird in Übereinstimmung mit dem o. a. Verfahren erhalten. Schmp.: 332-3 °C. (Wasser/Dioxan).

| | |
|---|---|
| Mikroanalyse C₁₅H₁₁NO₂ (237.25) | ber.: C 75.94, H 4.67, N 5.90; |
| | gef.: C 75.34, H 4.71, N 5.93. |

**Beispiel 5:** *5-Phenethyl-5,6,7,8-tetrahydro-indeno[1,2-b]indol-9,10-dion* **4d** Die Verbindung wird in Übereinstimmung mit dem o. a. Verfahren erhalten. Mp.: 189-90 °C. (Ethylacetat).

| | |
|---|---|
| Mikroanalyse C₂₃H₁₉NO₂ (341.40) | ber.: C 80.92, H 5.61, N 4.10; |
| | gef.: C 80.92; H 5.39; N 4.04. |

### Beispiel 6: 5-Phenethyl-5H-indeno[1,2,b]indol-6,9,10-trion 6d

Die Verbindung wird in Übereinstimmung mit dem o. a. Verfahren erhalten. Schmp:. > 310 °C. Zersetzung (Ethylacetat).

| | |
|---|---|
| Mikroanaylse C₂₃H₁₅NO₃ (353.11) | ber.: C 78.17, H 4.28, N 3.96; |
| | gef.: C 77.54, H 4.34, N 3.89. |

### Beispiel 7: DL-5-(1-Phenyl-ethyl)-5,6,7,8-tetrahydro-indeno[1,2-b]indol-9, 10-dion 4f

Die Verbindung wird in Übereinstimmung mit dem o. a. Verfahren erhalten. Schmp.:201 °C. (Ethylacetat).

| | |
|---|---|
| Mikroanalyse C₂₃H₁₉NO₂ (341.40) | ber.: C 80.92, H 5.61, N 4.10; |
| | gef.: C 80.79, H 5.74, N 4.00. |

### Beispiel 8: 5-[2-(3,4-Dimethoxy-phenyl)-ethyl]-5,6,7,8-tetrahydro-indeno[1,2-b]indol-9,10-dion

Die Verbindung wird in Übereinstimmung mit dem o. a. Verfahren erhalten Schmp.: 189-90 °C.( DMF/H₂0).

| | |
|---|---|
| Mikroanalyse C₂₅H₂₃NO₄ (401.45) | ber.: C 74.79, H 5.77, N 3.49; |
| | gef.: C 74.63, H 5.84, N 3.37. |

**Beispiel 9:** *5-Phenyl.5,6,7,8-tetrahydro-indeno[1,2-b]indol-9, 10-dion* **4g** Die Verbindung wird in Übereinstimmung mit dem o. a. Verfahren erhalten Schmp.: 288 °C. (Acetonitril).

| | |
|---|---|
| Mikroanalyse C₂₁H₁₅NO₂ (313.35) | ber.: C 80.49, H 4.82, N 4.47; |
| | gef.: C 80.49, H 4.78, N 4.43. |

### Beispiel 10: (9,10-Dioxo-6,8,9,10-tetrahydro-7H-indeno[1,2-b]indol-5-yl)-esigsäuremethylester

Die Verbindung wird in Übereinstimmung mit dem o. a. Verfahren erhalten M.p.: 256-7 °C (Ethylacetat)

| | |
|---|---|
| Mikroanalyse C₁₈H₁₅NO₄ (309.32) | ber.: C 69.89, H 4.89, N 4.53; |
| | gef.: C 69.61, H 4.92, N 4.50. |

### Beispiel 11: 5-(2-Dimethylamino-ethyl)-5,6,7,8-tetrahydro-indeno[1,2-b]indol-9,10-dion

Die Verbindung wird in Übereinstimmung mit dem o. a. Verfahren erhalten Schmp.: 178 °C.(Methanol).

| | |
|---|---|
| Mikroanalyse C₁₉H₂₀N₂O₂ (308.37) | ber: C 74.00, H 6.54, N 9.08; |
| | gef.: C 73.82, H 6.68, N 9.11. |

### Beispiel 12: 5-(3-Hydroxy-propyl)-5,6,7,8-tetrahydro-indeno[1,2-b]indol-9,10-dion

Die Verbindung wird in Übereinstimmung mit dem o. a. Verfahren erhalten Schmp.: 241 °C. (Acetonitril).

| | |
|---|---|
| Mikroanalyse C₁₈H₁₇NO₃ (295.33): | ber:. C 73.20, H 5,80, N 4.74; |
| | gef.: C 73.04, H 5.78, N 4.84. |

### Beispiel 13: (9,10-Dioxo-6,8,9,10-tetrahydro-7H-indeno[1,2-b]indol-5-y/)-essigsäure

Durch Verseifung mit methanolischem Kaliumhydroxyd der nach Beispiel 10 erhaltenen Verbindung
Schmp.: 279-80 °C. (Zers. ab 240 °C)(DMSO/H₂O).

| | |
|---|---|
| Mikroanalyse C₁₇H₁₃NO₄ (295.29): | ber.: C 69.15, H 4.44, N 4.74; |
| | gef.: C 69.11, H 4.55, N 4.74. |

### Beispiel 14: 5-(2-Hydroxy-ethyl)-5,6,7,8-tetrahydro-indeno[1,2-b]indol-9,10-dion

Die Verbindung wird in Übereinstimmung mit dem o. a. Verfahren erhalten
Schmp.: 262-3 °C.(DMSO/H₂O).

| | |
|---|---|
| Mikroanalyse C₁₇H₁₅NO₃ (281.31) | ber.: C 72.58, H 5.37, N 4.98; |
| | gef.: C 72.53, H 5.33, N 4.96. |

### Beispiel 15: 2-(9,10-Dioxo-6,8,9,10-tetrahydro-7H-indeno[1,2-b]indol-5-yl)-ethyl-methansulfonsäureester

Durch Umsetzung der nach Beispiel 14 erhaltenen Verbindung mit Methansulfonylchlorid
Schmp.: 230-1 °C. (Acetonitril).

| | |
|---|---|
| Mikroanalyse C₁₈H₁₇NO₅S (359.40): | ber.: C 60.15, H 4.77, N 3.90; |
| | gef.: C 60.25, H 4.84, N 4.03. |

### Beispiel 16: 5-Ethoxy-propyl-5,6,7,8-tetrahydro-indeno[1,2-b]indol-9,10-dion

Die Verbindung wird in Übereinstimmung mit dem o. a. Verfahren erhalten Schmp.: 129 °C. (Ethylacetat).

| | |
|---|---|
| Mikroanalyse C₂₀H₂₁NO₃ (323.39) | ber.: C 74.28, H 6.55, N 4.33; |
| | gef.: C 74.17, H 6.64, N 4.20. |

### Beispiel 17: 5-[2-(3,4-Dimethoxy-phenyl)-ethyl]-5,6,7,8-tetrahydro-indeno[1,2-b]indol-9,10-dion

Die Verbindung wird in Übereinstimmung mit dem o. a. Verfahren erhalten Schmp.: 189-90 °C.(DMF/H₂O).

| | |
|---|---|
| Mikroanalyse C₂₅H₂₃NO₄ (401.45): | ber.: C 74.79; H 5.77; N 3.49 |
| | gef.: C 74.63; H 5.84; N 3.37. |

### Beispiel 18: 5-(4-Methoxy-benzyl)-5,6,7,8-tetrahydro-indeno[1,2-b]indol-9,10-dion

Die Verbindung wird in Übereinstimmung mit dem o. a. Verfahren erhalten Schmp.: 198-9 °C.(Ethylacetat).

| | |
|---|---|
| Mikroanalyse C₂₃H₁₉NO₃ (357.40) | ber.: C 77.29, H 5.36, N 3.92; |
| | gef.: C 77.12, H 4.97, N 3.81. |

### Beispiel 19: 5-Isopropyl-7-methyl-5,6,7,8-tetrahydro-indeno[1,2-b]indol-9,10-dion

Die Verbindung wird in Übereinstimmung mit dem o. a. Verfahren erhalten Schmp.: 264-5 °C. (Ethylacetat).

| | |
|---|---|
| Mikroanalyse C₁₉H₁₉NO₂ (293.36) | ber.: C 77.79; H 6.53; N 4.77; |
| | gef.: C 77.55; H 6.73; N 5.06. |

### Beispiel 20: Methyl-7-methyl-9,10-dioxo-5-phenyl-5,6,7,8,9,10-hexahydro indeno[1,2-b]indol-8-carboxylat

Die Verbindung wird in Übereinstimmung mit dem o. a. Verfahren erhalten Schmp.: 249-50°C. (Ethylacetat).

| | |
|---|---|
| Mikroanalyse C₂₄H₁₉NO₄ (385.42) | ber.: C 74.79; H 4.97; N 3.63; |
| | gef.: C 74.58 ; H 5.21; N 3.51. |

### Beispiel 21: Methyl-7-methyl-9,10-dioxo-5,6,7,8,9,10-hexahydroindeno[1,2-b]indol-8-carboxylat

Die Verbindung wird in Übereinstimmung mit dem o. a. Verfahren erhalten. Schmp.: 287 °C. (DMSO/H₂O).

| | |
|---|---|
| Mikroanalyse C₁₈H₁₅NO₄ (309.32): | ber.: C 69.89; H 4.89; N 4.53; |
| | gef.: C 69.67; H 4.80; N 4.35. |

### Beispiel 22: 5-Benzyl-7-phenl-5,6,7,8-tetrahydro indeno[1,2-b]indol-9,10-dion

Die Verbindung wird in Übereinstimmung mit dem o. a. Verfahren erhalten. Schmp.: 252 °C. (Toluol).

| | |
|---|---|
| Mikroanalyse C₂₈H₂₁NO₂ (403.49) | ber.: C 83.35, H 5.25, N 3.47; |
| | gef.: C 83.54, H 5.27, N 3.33. |

### Beispiel 23: 5-Benzyl-7-phenyl-6,9-dihydro-5H-indeno[1,2-b]indol-6,9,10-trion

Die Verbindung wird in Übereinstimmung mit dem o. a. Verfahren erhalten Schmp.: 247-8 °C. (Acetonitril).
HRMS: C₂₈H₁₇NO₃ ber.: 415.12085, gef.: 415.12111.

### Beispiel 24: 7-Methyl-5-phenethyl-5,6,7,8-tetrahydro-indeno[1,2-b]indol-9.10-dion

Die Verbindung wird in Übereinstimmung mit dem o. a. Verfahren erhalten Schmp.: 228-9 °C. (Acetonitril).

| | |
|---|---|
| Mikroanalyse C₂₄H₂₁NO₂ (355.43): | ber.: C 81.10, H 5.96, N 3.94; |
| | gef.: C 80.85, H 6.07, N 3.98 |

### Beispiel 25: 2-(9-Hydroxy-10-oxo-10H-indeno[1,2-b]indol-5-yl)methansulfon-säureethylester

Die Verbindung wird in Übereinstimmung mit dem o. a. Verfahren erhalten Schmp.: 188 °C. (Acetonitril).

| | |
|---|---|
| Mikroanalyse C₁₈H₁₅NO₅S (357.38): | ber.: C 60.49, H 4.23, N 3.92; |
| | gef.: C 60.41, H 4.10, N 4.24. |

### Beispiel 26: 9-Hydroxy-5-isopropy/-5H-indeno[1,2-b]indol-10-on

Die Verbindung wird in Übereinstimmung mit dem o. a. Verfahren erhalten Schp.: 182 °C. (Methanol).

| | |
|---|---|
| Mikroanalyse C₁₈H₁₅NO₂ (277.32) | ber.: C 77.96, H 5.45, N 5.05; |
| | gef.: C 78.18, H 5.66, N 5.00. |

### Literatur zur Synthese der Edukte (hierin eingeschlossen durch Bezugnahme)

### Synthese cyclischer Enaminone:

Greenhill, J. V. et al.: J. Heterocyd. Chem. 1992, 29, 1375 -1383
Edafiogho, I. O. et al.: J. Med. Chem. 1992, 35, 2798 - 2805
Kesten, S. J. et al.: J. Med. Chem. 1992, 35, 3429 - 3447
Scott, K. R. et al. J. Med. Chem. 1993, 36, 1947 -1955 u. v. a.

### Synthese substituierter Indantrione:

Joullie, M. M. et al. Tetrahedron 1991, 47, 8791 - 8830
Hark, R. R. et al. : Can. J. Chem. 2009, 79, 1632 -1654
Hansen, D. B. et al.: Chem. Soc. Rev. 2005, 34, 408 - 417

### Deoxygenierungsreakion :

Synthese des Reagens nach Dorlars, A. in Houben-Weyl: Methoden der organischen Chemie Band 11/2, 4. Aufl., Seite 737

### Beispiel 27: IC₅₀-Werte ausgesuchter Verbindungen

**Tabelle 2: Hemmung rekombinanter humaner Proteinkinase CK2 (Holoenzym) durch ausgewählte synthetisierte substituierte Indeno[1,2-] indolderivate.**

| | Verbindung¹⁾ | R²⁾ | Hemmung % [10 µM]³⁾ | IC₅₀-Wert [µM]⁴⁾ |
|---|---|---|---|---|
| 1 | 4b | Iso-C₃H₇ | 93,4 | 0,11 |
| 2 | 4d | (CH₂)₂C₆H_{5'} | 67,0 | 0,82 |
| 3 | 4f | CH(CH₃)C₆H₅ | 66,0 | 4,66 |
| 4 | 4g | C₆H₅ | 60,0 | 1,44 |
| 5 | 6b | Iso-C₃H₇ | 73,3 | 5,05 |
| 6 | 6c | CH₂C₆H₅ | 60,0 | 1,49 |
| 7 | 6d | (CH₂)₂C₆H₅ | 70,0 | 5,74 |

| | | | | |
|---|---|---|---|---|
| ¹⁾ Bezeichnung entsprechend der Beispiele 1 - 26. ²⁾ R entspricht R in der Struktur I bzw. Struktur II, dabei sind R7 und R9 = H. ³⁾ Zum Testen der inhibitorischen Wirkung der synthetisierten substituierten Indeno[1,2-]indolderivate wurde die humane Proteinkinase CK2 in E. coli rekombinant exprimiert und bis zur Homogenität aufgereinigt. Die Expression und Reinigung der Proteinkinase CK2 und ihrer katalytischen Untereinheit erfolgte nach dem folgenden Protokoll in Anlehnung an Guerra et al. (25). Die α- und die β-Untereinheit der CK2 wurden entweder getrennt ausgehend vom pT7-7-System in BI21 (DE3) exprimiert oder zusammen ausgehend von dem bicistronischen Vektor pET11d-CK2α,β. Die Reinigungsprozedur war in beiden Fällen dieselbe. BL21 (DE3) wurden frisch mit den entsprechenden Konstrukten transformiert. Die entstandenen Kolonien wurden in eine Vorkultur überimpft und über Nacht bei 37°C bis zur Sättigung wachsen lassen. Am nächsten Tag wurden ausgehend von der Vorkultur eine Hauptkultur angelegt (6 I Medium/Konstrukt) und diese bis zu einer OD₆₀₀ₙₘ von 0.6 wachsen lassen. Die Expression des Proteins wurde dann durch den Zusatz von IPTG (1 mM) induziert. Die Induktion für CK2 α oder des Holoenzyms betrug 5-6 h bei 30°C, während die Induktion der CK2 β über 3 h bei 37°C lief. Die Bakterien wurden durch Zentrifugation bei 6000 g für 10 min geerntet. Die Pellets können bei -80°C aufgehoben werden. Alle Reinigungsschritte wurden in der Kälte durchgeführt, um eine Degradation des Enzyms zu vermeiden. Die Bakterien wurden zunächst in Wasser aufgenommen (100 ml/10 g Bakterien) und durch Ultraschall aufgeschlossen (3 x 30 s mit der Ultraschallsonde, unter Kühlung). Die Bakterienreste werden abzentrifugiert, der Überstand als wässriger Extrakt aufbewahrt. Das Bakterienpellet wurde nun in P1500-Puffer aufgenommen (100 ml/10 g) und über Nacht bei 4°C unter Rühren weiter extrahiert (P1500-Extrakt). Beide Untereinheiten aus beiden Extrakten wurden vereinigt und gegen P300 (20 mM Tris/HCl, pH 8.0, 0.3 M NaCl, 7 mM 2-Mercaptoethanol, 0.2 mM PMSF) dialysiert. Das Dialysat wurde auf eine P11-Kationenaustauschersäule (Phosphocellulose von Whatman, 150 ml gequollenes Gel) geladen, die zuvor mit P300 äquilibriert worden war. Die nachfolgende Chromatographie wurde mit Hilfe einer Chromatographieeinheit von Bio-Rad (Econo-System) durchgeführt. Die Säule wurde solange mit P300 gespült, bis die bei 280 nm gemessene Extinktion auf ihren Ausgangswert zurückfiel. Die an der Säule haftenden Proteine wurden durch das Anlegen eines linearen Salzgradienten zwischen 300 und 1500 mM NaCl eluiert und fraktioniert (80 Fraktionen ä 7.5 ml) aufgefangen (Flußrate 1.5 ml/min, Gesamtvolumen 600 ml). Die CK2-haltigen Fraktionen wurden anhand der Western Blot Analyse mit CK2-spezifischen Antikörpern und dem Aktivitätsassay mit dem synthetischen Peptid RRRDDDSDDD identifiziert. CK2 eluierte etwa in der Mitte des Gradienten mit 700 mM NaCl. Die Fraktionen wurden vereinigt, gegen P300 dialysiert und durch nochmaliges Laden auf eine P11-Matrix (ca. 15 ml gequollenes Gel) konzentriert. Die an die Säule bindenden Proteine wurden durch sofortiges Umstellen des Puffersystems auf P1500 in einem Volumen von 20-30 ml eluiert. Für die nachfolgende Gelfiltration an einer Superose 6-Matrix (Pharmacia, 2.6 x 90 cm) wurden die Fraktionen mit Hilfe von Polyethylenglykol auf ein Volumen von 5 ml eingeengt und anschließend gegen P1000 (20 mM Tris/HCl, pH 8.0, 1 M NaCl, 7 mM 2-Mercaptoethanol, 0.2 mM PMSF) dialysiert. Die Gelfiltration erfolgte mit P1000 mit einer Flußrate von 1 ml/min und wurde durch Messung der Extinktion bei 280 nm dokumentiert. Das Eluat wurde fraktioniert aufgefangen, wie oben beschrieben, analysiert, gegebenenfalls eingeengt und gegen P100 (20 mM Tris/HCl, pH 8.0, 100 mM NaCl, 7 mM 2-Mercaptoethanol, 0.2 mM PMSF) dialysiert. Das gereinigte Enzym wurde bei -80°C portioniert gelagert. Zur Testung wurden jeweils etwa 5 ng (= 5 U, wobei 1 U der Menge an Enzym entspricht, die 1 µmol Phosphat auf ein vorgegebenes Substrat überträgt) rekombinant exprimierte und aufgereinigte humane CK2 für 10 min bei Raumtemperatur mit einer Endkonzentration von 10 µM der Verbindung oder dem selben Volumen an DMSO als Kontrolle in Kinasepuffer (50 mM Tris/HCl, pH 7.5, 100 mM NaCl, 10 mM M₉Cl₂, 1 mM DTT) in einem Gesamtvolumen von 20 µl vorinkubiert. Die Verbindungen wurden gewöhnlich in einer Stammlösung von 5 mM in DMSO gelagert. Die Reaktion wurde durch Zugabe von 30 µl Assay Puffer (25 mM Tris/HCl, pH 8.5, 150 mM NaCl, 5 mM MgCl₂, 1 mM DTT, 100 µM ATP, 0.19 mM Substrat (Synthetisches Peptid RRRDDDSDDD) und 0.6 µCi [γ-³²]ATP) gestartet. Nach 15 min Inkubation bei 37°C wurde der gesamte Ansatz auf ein Ionenaustausch Chromatographie Filterpapier vom Typ P81 gespottet. Nach dreimaligem Waschen mit einem Überschuss an Phosphat (85 mM H₃PO₄) und danach mit Ethanol wurde der Filter getrocknet und die anheftende Menge an Radioaktivität in einem Szintillationszähler der Firma Packard bestimmt. Die anheftende Radioaktivität der Filter der DMSO Kontrollen wurde als 100 % Enzymaktivität der CK2 festgelegt und die Aktivität des Enzyms bei Zugabe und Vorinkubation mit den angegebenen Verbindungen in der Konzentration von 10 µM dazu ins Verhältnis gesetzt. Jeder Wert wurde mindestens dreimal in unabhängigen Experimenten bestimmt. ⁴⁾ Zur IC50-Wert Bestimmung wurde der Enzymtest wie unter ³⁾ beschrieben durchgeführt mit variablen Endkonzentrationen der Verbindungen im Konzentrationsbereich von 30 µM bis 0,01 µM in geeigneten Intervallen. Aus der so erstellten Eichkurve, die das lineare Verhältnis zwischen dem dekadischen Logarithmus der Verbindungskonzentration und der prozentualen Hemmung beschreibt, konnte der jeweilige IC₅₀-Wert berechnet werden. Jeder Wert wurde mindestens dreimal in unabhängigen Experimenten bestimmt. | | | | |

### Beispiel 28: Proliferation von Tumorzelllinien nach Inkubation mit verschiedenen Indeno[1,2-b]indolen.

Die IC₅₀-Werte der Zellproliferation wurde gemäß Bracht et al. (28) bestimmt. Die Ergebnisse sind in Tabelle 3 zusammengefasst.

### Beispiel 29: IC₅₀-Werte der Zellproliferation ausgesuchter Indeno[1,2,-b]indole für verschiedene Tumorzelllinien.

Die IC₅₀-Werte der Zellproliferation wurde gemäß Bracht et al. (28) bestimmt. Die Ergebnisse sind in Tabelle 4 zusammengefasst.

### Beispiel 30: IC₅₀-Werte [µM] ausgesuchter Indeno[1,2,-b]indole von verschiedenen humanen Proteinkinasen.

### a) Rekombinante Proteinkinasen

Die folgenden 24 Proteinkinasen wurden für die Bestimmung von Inhibitionsprofilen verwendet:
AKT1, ARK5, Aurora-A, Aurora-B, B-RAF-VE, CDK2/CycA, CDK4/CycD1, CK2-alpha1 (katalytische Untereinheit von CK2), EGF-R, EPHB4, ERBB2, FAK, IGF1-R, SRC, VEGF-R2, VEGF-R3, FLT3, INS-R, MET, PDGFR-beta, PLK1, SAK, TIE2, COT.

Alle Proteinkinasen wurden in Sf9-Insektenzellen als humane rekombinante GST-Fusionsproteine oder His-getaggte Proteine durch das Baculovirus-Expressionssystem exprimiert. Kinasen wurden durch Affinitätschromatography entweder durch GSH-Agarose (Sigma) oder Ni-NTH-Agarose (Qiagen) gereinigt. Die Reinheit der Kinasen wurde durch SDS-PAGE/Silberfärbung geprüft, und die Identität der Kinasen wurde durch Westernblot-Analyse mit spezifischen Antikörpern oder Massenspektroskopie verifiziert.

### b) Proteinkinasetest

Ein radiometrischer Proteinkinasetest (33PanQinase® Activity Assay) wurde zur Messung der Kinaseaktivität für die 24 Proteinkinasen verwendet. Die Kinasetests wurden in 96-Loch-FlashPlatesTM von Perkin Elmer/NEN (Boston, MA, USA) in einem Reaktionsvolumen von 50 µl durchgeführt. Die Reaktionsmischung wurde in 4 Schritten in der folgenden Reihenfolge pipettiert:
- 20 µl Testpuffer
- 5 µl ATP-Lösung (in H₂O)
- 5 µl Testverbindung (in 10 % DMSO)
- 10 µl Substrat /10 µl Enzymlösung (vorgemischt)

Für alle Enzyme enthielten die Tests 60 mM HEPES-NaOH, pH 7.5, 3 mM MgCl2, 3 mM MnCl2, 3 µM Na-Orthovanadat, 1.2 mM DTT, 50 µg/ml PEG₂₀₀₀₀, 1 µM [γ-33P]-ATP (etwa. 5 x 10⁵ cpm pro Loch).

Für die 24 Kinasen wurden die folgenden Mengen von Enzymen und Substrat pro Loch eingesetzt:

| # | *Kinase* | *Kinase* | Konz. | *Substrat* | *Substrat ng*/*50*µ*l* |
|---|---|---|---|---|---|
| | | *ng*/*50*µ*l* | | | |
| 1 | AKT1 | | 100 | GSK3(14-27) (Lot 006) | 1000 |
| 2 | ARK5 | | 100 | CHKtide (Lot 002) | 1000 |
| 3 | Aurora-A | | 50 | tetra(LRRWSLG) | 500 |
| 4 | Aurora-B | | 50 | tetra(LRRWSLG) | 250 |
| 5 | B-RAF VE | | 20 | MEK1 KM (Lot 018) | 250 |
| 6 | CDK2/CycA | | 100 | Histone H1 | 125 |
| 7 | CDK4/CycD1 | | 50 | Rb-CTF (Lot 011) | 500 |
| 8 | CK2-alpha1 | | 200 | Casein | 200 |
| 9 | EGF-R | | 25 | Poly(Glu,Tyr)4:1 | 125 |
| 10 | EPHB4 | | 10 | Poly(Glu, Tyr)4:1 | 125 |
| 11 | ERBB2 | | 100 | Poly(Glu, Tyr)4:1 | 125 |
| 12 | FAK | | 200 | Poly(Glu,Tyr)4:1 | 125 |
| 13 | IGF1-R | | 20 | Poly(Glu, Tyr)4:1 | 125 |
| 14 | SRC | | 10 | Poly(Glu, Tyr)4:1 | 125 |
| 15 | VEGF-R2 | | 10 | Poly(Glu,Tyr)4:1 | 125 |
| 16 | VEGF-R3 | | 100 | Poly(Glu,Tyr)4:1 | 125 |
| 8 | COT | | 400 | Autophosphorylation | - |
| 21 | PLK1 | | 50 | CHKtide (Lot 002) | 2000 |
| 22 | SAK | | 200 | p38-alphaKRKR (Lot 002) | 1000 |
| 23 | TIE2 | 006 | 200 | Poly(Glu,Tyr) 4:1 | 250 |
| 17 | FLT3 | SP007 | 100 | Poly(Ala,Glu,Lys,Tyr) 6:2:5:1 | 125 |
| 18 | INS-R | SP005 | 25 | Poly(Ala,Glu,Lys,Tyr) 6:2:5:1 | 125 |
| 19 | MET | SP011 | 100 | Poly(Ala,Glu,Lys,Tyr) 6:2:5:1 | 125 |
| 20 | PDGFR-beta | SP012 | 100 | Poly(Ala,Glu,Lys,Tyr) 6:2:5:1 | 125 |

Die Reaktionsmischung wurden bei 30° C für 80 Minuten inkubiert. Die Reaktion wurde mit 50 µl 2 % (v/v) H₃PO₄ gestoppt. Die Platten wurden abgesaugt und zweimal mit 200 µl 0.9 % (w/v) NaCl gewaschen. Der Einbau von ³³Pi wurde mit einem Mikrotiterplatten-Szintillationszähler (Microbeta Trilux, Wallac) bestimmt.

Alle Tests wurden mit einem BeckmanCoulter/Sagian-Robottersystem durchgeführt.

Die Ergebnisse sind in Tabelle 5 zusammengefasst.

### Tabellen

**Tabelle 1: Synthetisierte Indeno[1,2-b]Indole**

| | | | | |
|---|---|---|---|---|
| 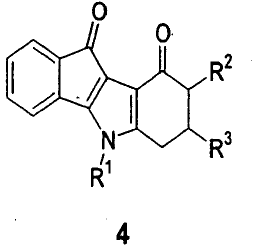 | | 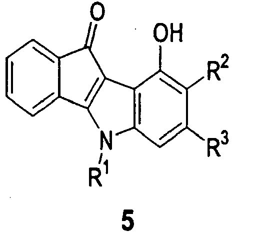 | 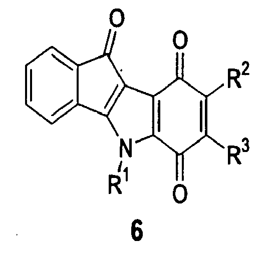 | |

| Zwischen produckte der Synthese | | | | |
|---|---|---|---|---|
| | **Compound** | **R¹** | **R²** | **R³** |
| **1** | **4a** | H | H | H |
| **2** | **4b** | *Iso*-C₃H₇ | H | H |
| **3** | **4c** | CH₂C₆H₅ | H | H |
| **4** | **4d** | (CH₂)₂C₆H₅ | H | H |
| **5** | **4e** | (CH₂)₃C₆H₅ | H | H |
| **6** | **4f** | CH(CH₃)C₆H₅ | H | H |
| **7** | **4g** | C₆H₅ | H | H |
| **8** | **4h** | CH₂-2-C₅H₄N | H | H |
| **9** | **4i** | CH₂c₆H₄-4-OCH₃ | H | H |
| **10** | **4j** | (CH₂)₂C₆H₃-3,4-(OCH₃)₂ | H | H |
| **11** | **4k** | CH₂C₆H₅ | H | (CH₃)₂ |
| **12** | **4l** | H | COOCH₃ | CH₃ |
| **13** | **4m** | CH₂C₆H₅ | COOCH₃ | CH₃ |
| **14** | **4n** | CH₂C₆H₅ | H | C₆H₅ |
| **15** | **4o** | CH₂C₆H₅ | H | CH₃ |
| **16** | **4p** | (CH₂)₂C₆H₅ | H | CH₃ |
| **17** | **5b** | *Iso*-C₃H₇ | H | H |
| **18** | **5c** | CH₂C₆H₅ | H | H |
| **19** | **5j** | (CH₂)₂C₆H₃-3,4-(OCH₃)₂ | H | H |
| **20** | **5n** | CH₂C₆H₅ | H | C₆H₅ |
| **21** | **6b** | *Iso*-C₃H₇ | H | H |
| **22** | **6c** | CH₂C₆H₅ | H | H |
| **23** | **6d** | (CH₂)₂C₆H₅ | H | H |

**Tabelle 2: IC₅₀-Werte ausgesuchter Indeno[1,2-b]indole für die humane Protein Kinase CK2**

| | **Substanz¹⁾** | **R²⁾** | **Hemmung % [10 µM]³⁾** | **IC₅₀ Wert [µM]⁴⁾** |
|---|---|---|---|---|
| **1** | **4b** | *Iso*-C₃H₇ | 93,4 | 0,11 |
| **2** | **4d** | (CH₂)₂C₆H₅ | 67,0 | 0,82 |
| **3** | **4f** | CH(CH₃)C₆H₅ | 66,0 | 4,66 |
| **4** | **4g** | C₆H₅ | 60,0 | 1,44 |
| **5** | **6b** | *Iso*-C₃H₇ | 73,3 | 5,05 |
| **6** | **6c** | CH₂C₆H₅ | 60,0 | 1,49 |
| **7** | **6d** | (CH₂)₂C₆H₅ | 70,0 | 5,74 |

| | | | | |
|---|---|---|---|---|
| ¹⁾ Bezeichnung entsprechend Tabelle 1 ²⁾ R entspricht R in der Struktur I bzw. Struktur II, dabei sind **R7 und R9 = H.** | | | | |

### Tabelle 3:

**Tabelle 3: Proliferation von Tumorzelllinien nach Inkubation mit verschiedenen Indeno[1,2 b]indole**

| **Verbindung** | | | **T/C (%)** | | | |
|---|---|---|---|---|---|---|
| | **5637** | | **SISO** | | **KYSE-70** | |
| | Mittelwert | sd | Mittelwert | sd | Mittelwert | sd |
| **4a** | **80,86** | 5,00 | **99,86** | 3,34 | **77,54** | 4,58 |
| **4b** | **99,05** | 9,73 | **100,02** | 2,13 | **74,87** | 3,00 |
| **4c** | **53,63** | 15,80 | **86,36** | 3,41 | **65,77** | 8,01 |
| **4d** | **47,97** | 16,44 | **65,49** | 7,76 | **67,46** | 10,05 |
| **4e** | **49,93** | 11,69 | **84,54** | 6,23 | **65,53** | 5,11 |
| **4f** | **64,86** | 17,41 | **95,82** | 2,24 | **62,30** | 10,58 |
| **4g**** | **86,33** | 16,49 | **100,85** | 0,68 | **81,77** | 5,93 |
| **4h** | **98,19** | 4,40 | **104,53** | 4,19 | **100,16** | 3,83 |
| **4i** | **78,20** | 25,23 | **93,63** | 5,14 | **79,24** | 21,34 |
| **4j** | **101,23** | 19,05 | **104,53** | 4,95 | **91,47** | 5,12 |
| **4k*** | **92,22** | 14,23 | **89,70** | 3,89 | **90,05** | 1,44 |
| **4l** | **79,09** | 20,73 | **103,81** | 13,20 | **98,85** | 12,38 |
| **4m** | **55,90** | 11,59 | **91,31** | 10,11 | **84,71** | 13,96 |
| **4n** | **39,38** | 7,50 | **80,94** | 23,98 | **75,20** | 22,27 |
| **4o** | **54,85** | 13,28 | **90,09** | 6,90 | **74,06** | 6,12 |
| **4p** | **49,75** | 13,06 | **96,33** | 10,31 | **72,12** | 5,30 |
| **5b** | **77,53** | 18,86 | **104,02** | 8,37 | **77,57** | 4,46 |
| **5c** | **58,02** | 7,63 | **94,16** | 4,84 | **74,80** | 5,80 |
| **5j**** | **104,23** | | **114,84** | | **90,07** | |
| **5n** | **19,81** | 23,69 | **59,42** | 27,96 | **41,13** | 25,86 |
| **6b** | **-1,73** | 1,07 | **0,28** | 1,50 | **1,26** | 3,50 |
| **6c** | **0,44** | 0,41 | **5,30** | 2,61 | **12,05** | 4,90 |
| **6d*** | **69,73** | 2,62 | **87,25** | 2,86 | **92,00** | 10,39 |

| | | | | | | |
|---|---|---|---|---|---|---|
| 1. Alle Substanzen wurden bei 20 µM getestet mit folgenden Ausnahmen: 2. * = 5 µM 3. ** = 10 µM **Blau markierte Substanzen wurden weiter untersucht.** | | | | | | |

**Tabelle 4: IC₅₀-Werte der Zellproliferationshemmung ausgesuchter Indeno[1,2-b]indole für verschiedene Tumorzellinien**

| **Verbindung** | | | | | | | | | **IC₅₀ (µM)** | | | | | | | |
|---|---|---|---|---|---|---|---|---|---|---|---|---|---|---|---|---|
| | **5637** | | **SISO** | | **KYSE-70** | | **MCF-7** | | **DAN-G** | | **L CLC** | | **A427** | | **RT-4** | |
| | MW | sd | MW | sd | MW | sd | MW | sd | MW | sd | MW | sd | MW | sd | MW | S d |
| **4d** | 10,71 | 5,01 | 12,26 | 2,28 | >20 | | 6,53 | 1,44 | >20 | | 5,66 | 2,94 | >20 | | >20 | |
| **6b** | 1,67 | 0,40 | 1,54 | 0,17 | 1,54 | 0,36 | 1,32 | 0,07 | >10 | | 1,47 | 0,19 | 1,40 | 0,22 | >10 | |
| **6c** | 2,75 | 0,59 | 2,82 | 0,56 | 2,83 | 0,30 | 3,27 | 0,40 | >20 | | 4,14 | 1,00 | 3,74 | 0.65 | >20 | |

| | | | | | | | | | | | | | | | | |
|---|---|---|---|---|---|---|---|---|---|---|---|---|---|---|---|---|
| **sd = Standardabweichung** | | | | | | | | | | | | | | | | |

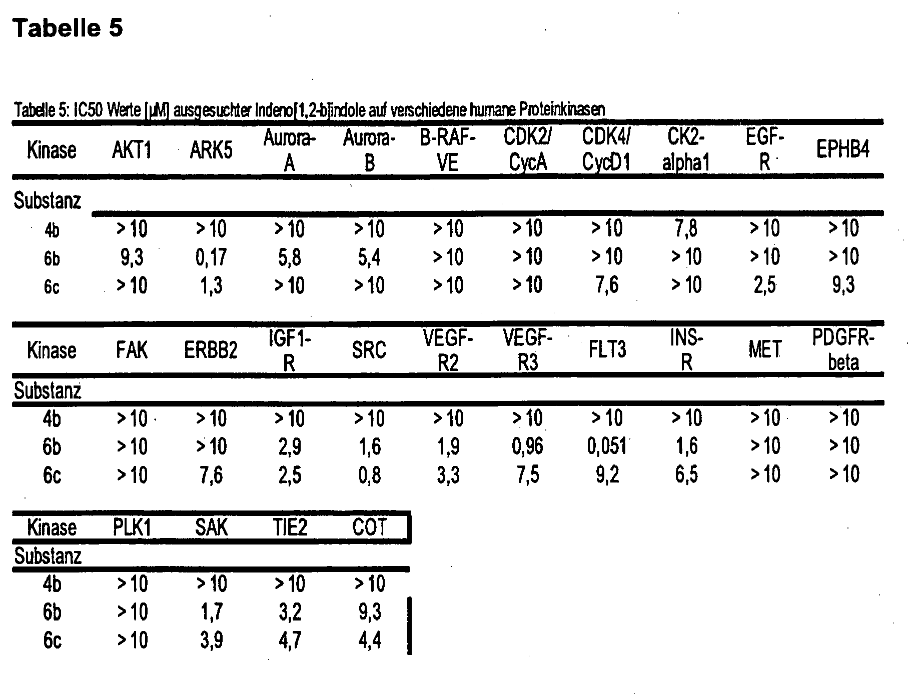

### Literatur

1. Buchanan, S. G., Protein structure: discovering selective protein kinase inhibitors. Targets 2003, 2, (3), 101-108.
2. Litchfield, D. W., Protein kinase CK2: structure, regulation and role in cellular decisions of life and death. Biochem J 2003, 369, (Pt 1), 1-15.
3. Bibby, A. C.; Litchfield, D. W., The Multiple Personalities of the Regulatory Subunit of Protein Kinase CK2: CK2 Dependent and CK2 Independent Roles Reveal a Secret Identity for CK2beta. Int J Biol Sci 2005, 1, (2), 67-79.
4. Meggio, F.; Pinna, L. A., One-thousand-and-one substrates of protein kinase CK2beta Faseb J 2003, 17, (3), 349-68.
5. Pepperkok, R.; Lorenz, P.; Ansorge, W.; Pyerin, W., Casein kinase II is required for transition of G0/G1, early G1, and G1/S phases of the cell cycle. J Biol Chem 1994, 269, (9), 6986-91.
6. Pepperkok, R.; Lorenz, P.; Jakobi, R.; Ansorge, W.; Pyerin, W., Cell growth stimulation by EGF: inhibition through antisenseoligodeoxynucleotides demonstrates important role of casein kinase II. Exp Cell Res 1991, 197, (2), 245-53.
7. Lorenz, P.; Pepperkok, R.; Ansorge, W.; Pyerin, W., Cell biological studies with monoclonal and polyclonal antibodies against human casein kinase II subunit beta demonstrate participation of the kinase in mitogenic signaling. J Biol Chem 1993, 268, (4), 2733-9.
8. Wang, G.; Ahmad, K. A.; Ahmed, K., Role of protein kinase CK2 in the regulation of tumor necrosis factor-related apoptosis inducing ligandinduced apoptosis in prostate cancer cells. Cancer Res 2006, 66, (4), 2242-9.
9. Tawfic, S.; Yu, S.; Wang, H.; Faust, R.; Davis, A.; Ahmed, K., Protein kinase CK2 signal in neoplasia. Histol Histopathol 2001, 16, (2), 573-82.
10. Hessenauer, A.; Montenarh, M.; Gotz, C., Inhibition of CK2 activity provokes different responses in hormone-sensitive and hormone-refractory prostate cancer cells. Int J Oncol 2003, 22, (6), 1263-70.
11. Yenice, S.; Davis, A. T.; Goueli, S. A.; Akdas, A.; Limas, C.; Ahmed, K., Nuclear casein kinase 2 (CK-2) activity in human normal, benign hyperplastic, and cancerous prostate. Prostate 1994, 24, (1), 11-6.
12. Landesman-Bollag, E.; Romieu-Mourez, R.; Song, D. H.; Sonenshein, G. E.; Cardiff, R. D.; Seldin, D. C., Protein kinase CK2 in mammary gland tumorigenesis. Oncogene 2001, 20, (25), 3247-57.
13. Daya-Makin, M.; Sanghera, J. S.; Mogentale, T. L.; Lipp, M.; Parchomchuk, J.; Hogg, J. C.; Pelech, S. L., Activation of a tumor-associated protein kinase (p40TAK) and casein kinase 2 in human squamous cell carcinomas and adenocarcinomas of the lung. Cancer Res 1994, 54, (8), 2262-8.
14. Faust, R. A.; Gapany, M.; Tristani, P.; Davis, A.; Adams, G. L.; Ahmed, K., Elevated protein kinase CK2 activity in chromatin of head and neck tumors: association with malignant transformation. Cancer Lett 1996, 101, (1), 31-5.
15. ole-MoiYoi, O. K.; Brown, W. C.; lams, K. P.; Nayar, A.; Tsukamoto, T.; Macklin, M. D., Evidence for the induction of casein kinase II in bovine lymphocytes transformed by the intracellular protozoan parasite Theileria parva. Embo J 1993, 12, (4), 1621-31.
16. Seldin, D. C.; Leder, P., Casein kinase II alpha transgene-induced murine lymphoma: relation to theileriosis in cattle. Science 1995, 267, (5199), 894-7.
17. Scaglioni, P. P.; Yung, T. M.; Cai, L. F.; Erdjument-Bromage, H.; Kaufman, A. J.; Singh, B.; Teruya-Feldstein, J.; Tempst, P.; Pandolfi, P. P., A CK2-dependent mechanism for degradation of the PML tumor suppressor. Cell 2006, 126, (2), 269-83.
18. Sarno, S.; Ruzzene, M.; Frascella, P.; Pagano, M. A.; Meggio, F.; Zambon, A.; Mazzorana, M.; Di Maira, G.; Lucchini, V.; Pinna, L. A., Development and exploitation of CK2 inhibitors. Mol Cell Biochem 2005, 274, (1-2), 69-76.
19. Sarno, S.; Salvi, M.; Battistutta, R.; Zanotti, G.; Pinna, L. A., Features and potentials of ATP-site directed CK2 inhibitors. Biochim Biophys Acta 2005, 1754, (1-2), 263-70.
20. Yim, H.; Lee, Y. H.; Lee, C. H.; Lee, S. K., Emodin, an anthraquinone derivative isolated from the rhizomes of Rheum palmatum, selectively inhibits the activity of casein kinase II as a competitive inhibitor. Planta Med 1999, 65, (1), 9-13.
21. Sarno, S.; Reddy, H.; Meggio, F.; Ruzzene, M.; Davies, S. P.; Donella-Deana, A.; Shugar, D.; Pinna, L. A., Selectivity of 4,5,6,7-tetrabromobenzotriazole, an ATP site-directed inhibitor of protein kinase CK2 ('casein kinase-2'). FEBS Lett 2001, 496, (1), 44-8.
22. Pagano, M. A.; Meggio, F.; Ruzzene, M.; Andrzejewska, M.; Kazimierczuk, Z.; Pinna, L. A., 2-Dimethylamino-4,5,6,7-tetrabromo-1H-benzimidazole: a novel powerful and selective inhibitor of protein kinase CK2. Biochem Biophys Res Commun 2004, 321, (4), 1040-4.
23. Sarno, S.; de Moliner, E.; Ruzzene, M.; Pagano, M. A.; Battistutta, R.; Bain, J.; Fabbro, D.; Schoepfer, J.; Elliott, M.; Furet, P.; Meggio, F.; Zanotti, G.; Pinna, L. A., Biochemical and three-dimensional-structural study of the specific inhibition of protein kinase CK2 by [5-oxo-5,6-dihydroindolo-(1,2-a)quinazolin-7-yl]acetic acid (IQA). Biochem J 2003, 374, (Pt 3), 639-46.
24. Cozza, G.; Bonvini, P.; Zorzi, E.; Poletto, G.; Pagano, M. A.; Sarno, S.; Donella-Deana, A.; Zagotto, G.; Rosolen, A.; Pinna, L. A.; Meggio, F.; Moro, S., Identification of ellagic acid as potent inhibitor of protein kinase CK2: a successful example of a virtual screening application. J Med Chem 2006, 49, (8), 2363-6.
25. Guerra, B.; Gotz, C.; Wagner, P.; Montenarh, M.; Issinger, O. G., The carboxy terminus of p53 mimics the polylysine effect of protein kinase CK2-catalyzed MDM2 phosphorylation. Oncogene 1997, 14, (22), 2683-8.
26. Yamada et al, PNAS 2005, 102:7736-7741
27. Schneider et al., LUPUS 2007, 16:221-226
28. Bracht et al. Anti-Cancer Drugs 2006, 17:41-51
29. Hemmerling et al. Z. Naturforsch. 59B, 2006, 1143-1152

## Patentansprüche

1. Verbindung der Struktur I, Struktur IA oder (Struktur II 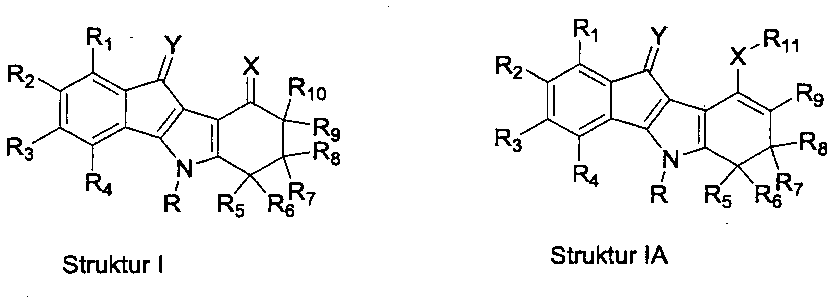 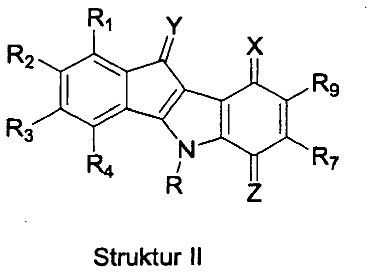 in der:
**R** bedeutet
ein **Wasserstoffatom** oder
eine geradkettige oder verzweigte **C₁- C₈-Alkylgruppe** oder
eine **C₃- C₈-Cycloalkylgruppe** oder
eine geradkettige oder verzweigte **C₂-C₈-Alkenylgruppe** oder
eine **C₃- C₈-Cycloalkenylgruppe** oder
eine **Arylgruppe** oder
eine **Heteroarylgruppe** oder
eine **Aryl-C₁-C₈-Alkylgruppe** oder
eine **Heteroaryl-C₁-C₈-Alkylgruppe,**
wobei **R** gegebenenfalls einfach oder mehrfach, gleich oder verschieden substituiert ist durch
Hydroxygruppen oder
Cyanogruppen oder
Nitrogruppen oder
Halogenatome oder
Carboxygruppen oder
Sulfonsäuregruppen oder
Gruppen der Formel COOR₁₂ (Alkoxycarbonylgruppe) oder SO₂OR₁₃ (Alkoxysulfonsäuregruppe), in denen R₁₂, R₁₃, die gleich oder verschieden sind, jeweils eine geradkettige oder verzweigte C₁-C₈-Alkylgruppe oder C₃-C₈-Cycloalkylgruppe oder eine geradkettige oder verzweigte C₂-C₈-Alkenylgruppe bedeutet, die gegebenenfalls einfach oder mehrfach gleich oder verschieden substituiert ist durch Hydroxygruppen, Cyanogruppen, Nitrogruppen, Halogenatome, Carboxygruppen oder Gruppen der Formeln NR₁₄R₁₅, CONR₁₄R₁₅, CSNR₁₄R₁₅, SO₂NR₁₄R₁₅, in denen R₁₄, R₁₅, die gleich oder verschieden sind, jeweils ein Wasserstoffatom oder eine geradkettige oder verzweigte C₁-C₈-Alkylgruppe oder eine C₃-C₈-Cycloalkylgruppe bedeuten oder die gemeinsam mit dem sie tragenden Stickstoffatom einen stickstoffhaltigen Heterocyclus bilden, oder
Gruppen der Formeln CONR₁₆R₁₇ (Carboxamidogruppe), CSNR₁₆R₁₇ (Thiocarboxamidogruppe), SO₂NR₁₆R₁₇ (Sulfonamidgruppen), in denen R₁₆, R₁₇, die gleich oder verschieden sind, jeweils ein Wasserstoffatom oder eine geradkettige oder verzweigte C₁ - C₈-Alkylgruppe oder C₃ - C₈-Cycloalkylgruppe oder eine geradkettige oder verzweigte C₂ - C₈-Alkenylgruppe bedeuten oder gemeinsam mit dem sie tragenden Stickstoffatom einen stickstoffhaltigen Heterocyclus bilden, und die gegebenenfalls einfach oder mehrfach gleich oder verschieden substituiert sind durch Hydroxygruppen, Cyanogruppen, Nitrogruppen, Halogenatome, Carboxygruppen, oder
Aminogruppen NR₁₈R₁₉, in denen R₁₈ R₁₉, die gleich oder verschieden sind, jeweils ein Wasserstoffatom oder eine geradkettige oder verzweigte C₁ - C₈-Alkylgruppe oder C₃ - C₈-Cycloalkylgruppe oder geradkettige oder verzweigte C₂ - C₈-Alkenylgruppe bedeuten oder die gemeinsam mit dem sie tragenden Stickstoffatom eine stickstoffhaltigen Heterocyclus bilden, oder
Acyloxygruppen OCOR₂₀, in denen R₂₀ eine geradkettige oder verzweigte C₁ - C₈-Alkylgruppe oder C₃ - C₈-Cycloalkygruppe oder eine geradkettige oder verzweigteC₂ - C₈-Alkenylgruppe bedeutet, die gegebenenfalls gleich oder verschieden, einfach oder mehrfach substituiert ist durch Hydroxygruppen, Cyanogruppen, Nitrogruppen, Halogenatome, Carboxygruppen, oder Gruppen der Formeln COOR₂₁, NR₂₁R₂₂, CONR₂₁R₂₂, CSNR₂₁R₂₂, SO₂NR₂₁R₂₂, in denen R₂₁, R₂₂, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bilden, oder
Gruppen der Formel OR₂₃NR₂₄R₂₅ (Aminoalkyloxy), in denen R₂₃ eine geradkettige oder verzweigte C₁- C₈-Alkylgruppe, R₂₄, R₂₅, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bilden,
wobei **R₁, R₂, R₃, R₄, R₅, R₆, R₇, R₈, R₉, R₁₀** die gleich oder verschieden sind, jeweils unabhängig voneinander bedeuten
ein **Wasserstoffatom** oder
eine geradkettige oder verzweigte C₁ - C₈-**Alkylgruppe** oder
eine C₃ - C₈-**Cycloalkylgruppe** oder
eine geradkettige oder verzweigte C₂ - C₈-**Alkenylgruppe** oder
eine C₃ - C₈-**Cycloalkenylgruppe** oder
eine **Arylgruppe** oder
eine geradkettige oder verzweigte **Aryl**-C₁ - C₈**-Alkylgruppe** oder
eine **Heteroarylgruppe** oder
eine **Hydroxygruppe** oder
eine geradkettige oder verzweigte C₁ - C₈-**Alkoxygruppe** oder
eine C₃ - C₈-**Cycloalkoxygruppe** oder
eine geradkettige oder verzweigte C₂- C₈-**Alkenyloxygruppe** oder
eine C₃ - C₈-**Cycloalkenyloxygruppe** oder
eine C₁ - C₂-**Alkylendioxygruppe**, die eine der Gruppen R₁ - R₁₀ mit einer benachbarten Gruppe R₁ - R₁₀ bildet, oder
eine **Acyloxygruppe OCOR₂₆**, in der R₂₆ eine geradkettige oder
verzweigte C₁ - C₈-Alkylgruppe oder eine geradkettige oder
verzweigte C₂- C₈-Alkenylgruppe oder eine C₃ - C₈-Cycloalkygruppe bedeutet, oder
eine geradkettige oder verzweigte **Aryl**-C₁ - C₈-**Alkoxygruppe** oder
eine **Alkoxycarbonylalkylgruppen R₂₇COoR₂₈,** in denen R₂₇, R₂₈, die gleich oder verschieden sind, jeweils eine geradkettige oder
verzweigte C₁ - C₈-Alkylgruppe oder eine C₃ - C₈-Cycloalkygruppe oder eine geradkettige oder verzweigte C₂ - C₈-Alkenylgruppe sind, oder
eine **Carboxygruppe, COOH,** oder
eine **Gruppe der Formeln CONR₂₉R₃₀ (Carboxamidogruppe), CSNR₂₉R₃₀ (Thiocarboxamidogruppe), SO₂NR₂₉R₃₀ (Sulfonamidgruppe),** in denen R₂₉, R₃₀, die gleich oder verschieden sind, jeweils ein Wasserstoffatom oder eine geradkettige oder
verzweigte C₁ - C₈-Alkylgruppe oder C₃ - C₈-Cycloalkylgruppe bedeuten oder gemeinsam mit dem sie tragenden Stickstoffatom einen stickstoffhaltigen Heterocyclus bilden, oder
eine **Alkoxycarbonylgruppen COOR₃₁,** in denen R₃₁ eine geradkettige oder verzweigte C₁ - C₈-Alkylgruppe oder eine C₃ - C₈-Cycloalkygruppe oder eine geradkettige oder verzweigte C₂ - C₈-Alkenylgruppe ist, oder
eine **Aminogruppe NR₃₂R₃₃,** in der R₃₂, R₃₃, die gleich oder verschieden sind, jeweils ein Wasserstoffatom oder eine geradkettige oder verzweigte C₁ - C₈-Alkylgruppe oder C₃ - C₈-Cycloalkylgruppe, eine geradkettige oder verzweigte C₂-C₈-Alkenylgruppe bedeuten oder die gemeinsam mit dem sie tragenden Stickstoffatom einen stickstoffhaltigen Heterocyclus bilden, oder
eine **Aminoalkyloxygruppe** der Formel **OR₃₄NR₃₅R₃₆,** in der R₃₄ eine geradkettige oder verzweigte C₁- C₈-Alkylgruppe oder eine C₃ - C₈-Cycloalkygruppe oder eine geradkettige oder verzweigte C₂ - C₈ -Alkenylgruppe bedeutet, R₃₅, R₃₆, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bilden,
wobei jede der Gruppen **R₁, R₂, R₃, R₄, R₅, R₆, R₇, R₈, R₉, R₁₀** gegebenenfalls einfach oder mehrfach, gleich oder verschieden unabhängig voneinander substituiert ist durch
Hydroxygruppen oder
Cyanogruppen oder
Nitrogruppen oder
Halogenatome oder
Carboxygruppen oder
Sulfonsäuregruppen oder
Arylgruppe oder
Heteroarylgruppe oder
Gruppen der Formeln COOR₃₇, NR₃₇R₃₈, CONR₃₇R₃₈ (Carboxamidogruppe), CSNR₃₇R₃₈ (Thiocarboxamidogruppe), SO₂NR₃₇R₃₈ (Sulfonamidgruppe), in denen R₃₇, R₃₈, die gleich oder verschieden sind, jeweils ein Wasserstoffatom oder eine geradkettige oder verzweigte C₁ - C₈-Alkylgruppe oder C₃ - C₈-Cycloalkylgruppe bedeuten oder gemeinsam mit dem sie tragenden Stickstoffatom einen stickstoffhaltigen Heterocyclus bilden, und die gegebenenfalls einfach oder mehrfach gleich oder verschieden substituiert sind durch Hydroxygruppen, Cyanogruppen, Nitrogruppen, Halogenatome, Carboxygruppen oder Gruppen der Formeln COOR₃₉, NR₃₉R₄₀, CONR₃₉R₄₀, CSNR₃₉R₄₀, SO₂NR₃₉R₄₀, in denen R₃₉, R₄₀, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bilden,
wobei **X, Y, Z,** die gleich oder verschieden sind, jeweils unabhängig voneinander bedeuten
ein **Sauerstoffatom**
eine **Iminofunktion** NR₄₁, in der R₄₁ ein Wasserstoffatom oder eine lineare oder verzweigte C₁ - C₈-Alkylgruppe oder eine C₃ - C₈-Cycloalkylgruppe oder eine lineare oder verzweigte C₂ - C₈-Alkenylgruppe oder eine C₃ - C₈-Cycloalkenylgruppe oder eine Arylgruppe oder Heteroarylgruppe bedeutet, oder
eine **Alkyloximinofunktion** NOR₄₂, in der R₄₂ ein Wasserstoffatom oder eine lineare oder verzweigte C₁ - C₈-Alkylgruppe oder eine C₃ - C₈-Cycloalkylgruppe oder eine lineare oder verzweigte C₂ - C₈-Alkenylgruppe oder eine C₃ - C₈-Cycloalkenylgruppe bedeutet, oder
eine **Aryloximinofunktion** NOR₄₃, in der R₄₃ eine Arylgruppe oder Heteroarylgruppe bedeutet,
wobei NR₄₁, NOR₄₂, und NOR₄₃, gegebenfalls einfach oder mehrfach, identisch oder verschieden unabhängig voneinander substituiert sind durch Arylgruppen, Heteroarylgruppen, Hydroxygruppen, Cyanogruppen, Nitrogruppen, Halogenatome oder durch Gruppen der Formeln COOR₄₄, NR₄₄R₄₅ (Aminogruppe), CONR₄₄R₄₅ (Carboxamidogruppe), CSNR₄₄R₄₅ (Thiocarboxamidogruppe), SO₂NR₄₄R₄₅ (Sulfonamidgruppe), in denen R₄₄, R₄₅, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten, oder die gegebenenfalls gemeinsam mit dem sie tragenden Stickstoffatom einen stickstoffhaltigen Heterocyclus bilden,
wobei **X-R₁₁** in Struktur IA bedeutet
eine **Hydroxygruppe** oder
eine **Alkoxygruppe,** in der R₁₁ eine geradkettige oder verzweigte C₁ - C₈-Alkylgruppe oder eine C₃ - C₈-Cycloalkylgruppe oder eine geradkettige oder verzweigte C₂ - C₈-Alkylengruppe ist, die gegebenenfalls einfach oder mehrfach substituiert ist durch Arylgruppen, Heteroarylgruppen, Hydroxygruppen, Cyanogruppen, Nitrogruppen, Halogenatome, Carboxygruppen oder Gruppen der Formeln COOR₄₆, NR₄₆R₄₇, CONR₄₆R₄₇, CSNR₄₆R₄₇, SO₂NR₄₆R₄₇, in denen R₄₆, R₄₇, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bildet, oder
eine **Acyloxygruppe** OCOR₄₈, in der R₄₈ eine geradkettige oder verzweigte C₁ - C₈-Alkylgruppe oder eine geradkettige oder verzweigte C₂ - C₈-Alkenylgruppe oder eine C₃ - C₈-Cycloalkylgruppe oder eine Arylgruppe oder eine Heteroarylgruppe bedeutet, die gegebenenfalls einfach oder mehrfach substituiert ist durch Arylgruppen, Heteroarylgruppen, Hydroxygruppen, Cyanogruppen, Nitrogruppen, Halogenatome, Carboxygruppen oder Gruppen der Formeln COOR₄₉, NR₄₉R₅₀, CONR₄₉R₅₀, CSNR₄₉R₅₀, SO₂NR₄₉R₅₀, in denen R₄₉, R₅₀, die gleich oder verschieden sein können, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bildet,
wobei unter **C₂ - C₈-Alkenylgruppen** jeweils unabhängig voneinander geradkettige oder verzweigte Kohlenwasserstoffketten zu verstehen sind, die mindestens eine bis zur maximalen Anzahl konjugierter Doppelbindungen enthalten, und
wobei unter **C₃ - C₈-Cycloalkenylgruppen** jeweils unabhängig voneinander ringförmige Kohlenwasserstoffe zu verstehen sind, die mindestens eine bis zur maximalen Anzahl konjugierter Doppelbindungen enthalten, und
wobei unter **Arylgruppen** jeweils unabhängig voneinander Phenyl oder Naphthylgruppen zu verstehen sind, die mit einem oder mehreren identischen oder verschiedenen Substituenten, wie linearen oder verzweigten C₁ - C₈-Alkylgruppen, C₃ - C₈-Cycloalkylgruppen, Phenyl-C₁-C₈-alkylgruppe, Naphthyl-C₁-C₈-alkylgruppe,C₁-C₂-Alkylendioxygruppe, Hydroxygruppe, Nitrogruppe, Cyanogruppe, Halogenatome, linearen oder verzweigten C₁ - C₈-Alkoxygruppen, Phenoxygruppe, linearen oder verzweigten C₁ - C₈-Alkoxycarbonygruppen, Carboxygruppe, Gruppen der Formeln OCR₅₁NR₅₂R₅₃ (Amino-C₁ - C₈-alkoxygruppe), NR₅₂R₅₃ (Aminogruppe), CONR₅₂R₅₃ (Carboxamidogruppe), CSNR₅₂R₅₃ (Thiocarboxamidogruppe), SO₂NR₅₂R₅₃ (Sulfonamidgruppe), in denen R₅₁ eine geradkettige oder verzweigte C₁ - C₈-Alkylgruppe bedeutet, R₅₂, R₅₃, die gleich oder verschieden sind, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bilden, substituiert sein können,
wobei unter **Heteroarylgruppen** jeweils unabhängig voneinander aromatische mono- oder bicyclische Ringsysteme mit 5 bis 12 Ringgliedern, die 1 - 3 Heteroatome (O, N, S) enthalten, zu verstehen sind, die mit einem oder mehreren identischen oder verschiedenen Substituenten wie linearen oder verzweigten C₁ - C₈-Alkylgruppen, C₃ - C₈-Cycloalkylgruppen, linearen oder verzweigten C₁ - C₈-Alkylengruppen, Arylgruppen, Hydroxygruppen, Nitrogruppen, Cyanogruppen, Halogenatome, linearen oder verzweigten C₁ - C₈-Alkoxygruppen, C₃ - C₈-Cycloalkoxygruppen, Phenoxygruppen, Gruppen der Formeln OCR5₄NR₅₅R₅₆ (Amino-C₁ - C₈-alkoxygruppe), NR₅₅R₅₆ (Aminogruppe), CONR₅₅R₅₆ (Carboxamidogruppe), CSNR₅₅R₅₆ (Thiocarboxamidogruppe), SO₂NR₅₅R₅₆ (Sulfonamidgruppe), in denen R₅₄ eine geradkettige oder verzweigte C₁ - C₈-Alkylgruppe bedeutet, R₅₅, R₅₆ die gleich oder verschieden sein können, jeweils ein Wasserstoffatom oder eine geradkettige oder verzweigte C₁ - C₈-Alkylgruppe oder eine C₃ - C₈-Cycloalkylgruppe bedeuten oder die gemeinsam mit dem sie tragenden Stickstoffatom einen stickstoffhaltigen Heterocyclus bilden, substituiert sein können, und
wobei unter einem stickstoffhaltigen **Heterocyclus** jeweils unabhängig voneinander ein gesättigter Monocyclus mit 5 - 7 Ringglieder zu verstehen ist, der neben Stickstoff 1 - 3 Heteroatome (vorzugsweise O,N,S) enthält, insbesondere Pyrrolidinyl-, Piperidyl-, Morpholinyl- und Piperazinylgruppen,
oder ein Salz davon mit einer pharmazeutisch gebräuchlichen Säure, wobei unter einer **pharmazeutisch gebräuchliche Säuren** insbesondere Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Weinsäure, Zitronensäure, Ascorbinsäure, Oxalsäure zu verstehen ist,
oder ein Isomerengemisch oder ein reines Isomer davon, wobei unter **Isomeren** optische Isomere und geometrische Isomere an Doppelbindungen zu verstehen sind, insbesondere Diastereomere Enantiomer und E/Z-Isomere.

2. Verbindung nach Anspruch 1, in der R bedeutet
ein **Wasserstoffatom** oder
eine geradkettige oder verzweigte **C₁-C₈-Alkylgruppe** oder
eine **C₃-C₈-Cycloalkylgruppe** oder
eine geradkettige oder verzweigte **C₂-C₈-Alkenylgruppe** oder
eine **C₃-C₈-Cycloalkenylgruppe** oder
eine **Arylgruppe** oder
eine **Heteroarylgruppe,** oder
eine **Aminogruppe NR₃₂R₃₃,** oder
eine geradkettige oder verzweigte C₁ - C₈-**Alkoxygruppe** oder
eine C₃-C₈-**Cycloalkoxygruppe**,
welche wie in Anspruch 1 definiert sind und gegebenenfalls wie in Anspruch 1 definiert substituiert sind.

3. Verbindung nach Anspruch 1 oder 2, worin **R₁, R₂, R₃, R₄, R₅, R₆, R₇, R₈, R₉, R₁₀** die gleich oder verschieden sind, jeweils unabhängig voneinander bedeuten
ein **Wasserstoffatom** oder
eine geradkettige oder verzweigte C₁ - C₈-**Alkylgruppe** oder
eine C₃ - C₈-**Cycloalkylgruppe** oder
eine geradkettige oder verzweigte C₂ - C₈-**Alkenylgruppe** oder
eine C₃ - C₈-**Cycloalkenylgruppe** oder
eine **Arylgruppe** oder
eine **Heteroarylgruppe** oder
eine **Hydroxygruppe** oder
eine **Acyloxygruppe OCOR₂₆,**
eine **Carboxygruppe COOH,** oder
eine **Aminogruppe NR₃₂R₃₃,** oder
eine geradkettige oder verzweigte C₁ - C₈-**Alkoxygruppe** oder
eine C₁ - C₂-**Alkylendioxygruppe**,
welche wie in Anspruch 1 definiert sind und gegebenenfalls wie in Anspruch 1 definiert substituiert sind.

4. Verbindung nach einem der Ansprüche 1 bis 3, in der X ein Sauerstoffatom ist, und/oder
Y ein Sauerstoffatom ist, und/oder
Z ein Sauerstoffatom ist.

5. Verbindung nach einem der Ansprüche 1 bis 3, in der R₅ - R₁₀ Wasserstoff sind, und/oder
R₇ und R₉ Wasserstoff sind.

6. Verbindung nach einem der Ansprüche 1 bis 3, in der R₁-R₁₀ identisch oder verschieden sind und Wasserstoff, eine geradkettige oder verzweigte C₁ - C₈-Alkyl- oder eine geradkettige oder verzweigte C₁ - C₈-Alkoxygruppen sind.

7. Verbindung nach einem der Ansprüche 1 bis 3, in der R ein Wasserstoffatom oder eine linear oder verzweigte C₁ - C₈ -Alkylgruppe oder eine Arylgruppe wie in Anspruch 1 definiert oder eine linear oder verzweigte Aryl-C₁ - C₈ -Alkylgruppe wie in Anspruch 1 definiert ist.

8. Verbindung nach einem der Ansprüche 1 bis 3 mit der Struktur I mit der IUPAC-Bezeichnung
*5-Isopropyl-5,6,7 8-tetrahydro-indeno[1,2-b]indol-9,10-dion,*
*5-Phenethyl-5,6,7,8-tetrahydro-indeno[1,2-b]indol-9,10-dion,*
*5-(1-Phenyl-ethyl)-5,6,7,8-tetrahydro-indeno[1,2-b]indol-9,10-dion,* ihrer Isomeren oder
*5-Phenyl-5,6,7,8-tetrahydro-indeno[1,2-b]indol-9,10-dion,*
oder ein Salz davon mit einer pharmazeutisch gebräuchlichen Säure.

9. Verbindung nach einem der Ansprüche 1 bis 3 mit der Struktur II mit der IUPAC-Bezeichnung
*5-Isopropyl-5H-indeno[1,2-b]indol-6,9,10-trion,*
*5-Benzyl-5,6,9,10-tefrahydroindeno[1,2-b]indol-6,9,10-trion,* oder
*5-Phenethyl-5H-indeno[1,2-b]indol-6,9,10-trion,*
oder ein Salz davon mit einer pharmazeutisch gebräuchlichen Säure.

10. Verbindung nach einem der Ansprüche 1 bis 3 mit der Struktur IA, wobei XR₁₁ OR₁₁ ist und wobei R₁₁ wie in Anspruch 1 definiert ist.

11. Verwendung einer Verbindung nach einem der Ansprüche 1 bis 10 zur Hemmung einer Proteinkinase *in vitro,*
wobei die Verbindung nach einem der Ansprüche 1 - 10 gegebenenfalls in Kombination mit einem weiteren Proteinkinasehemmstoff eingesetzt wird.

12. Verwendung einer Verbindung nach einem der Ansprüche 1 bis 10 zur Herstellung eines Medikamentes zur Behandlung von Krebs oder/und einem Tumor, oder zur Herstellung eines Diagnostikums für Krebs und/oder eine Tumorerkrankung.

13. Verwendung einer Verbindung nach einem der Ansprüche 1 bis 10 zur Herstellung eines Medikamentes zur Behandlung von einer entzündlichen Erkrankung, oder zur Herstellung eines Diagnostikums für eine entzündliche Erkrankung.

14. Pharmazeutische Zubereitung,
**dadurch gekennzeichnet,**
**dass** sie als Wirkstoff wenigstens eine Verbindung der Struktur I,
Struktur IA oder Struktur II nach einem der Ansprüche 1 bis 10 in einer wirksamen Dosierung enthält und gegebenenfalls mit wenigstens einem pharmazeutisch gebräuchlichen Zusatz oder/und Hilfsstoff bereitet wurde, gegebenenfalls wenigstens einen weiteren Wirkstoff umfasst.

15. Verfahren zur Synthese einer Verbindung der Struktur I nach einem der Ansprüche 1 bis 10, umfassend die Schritte:
(1) Herstellung eines cyclischen Enaminons **2** aus einem cyclischen 1,3-Diketon durch Reaktion mit Ammoniak oder/und einem primären Amin und Bereitstellen eines Indantrions **1,** 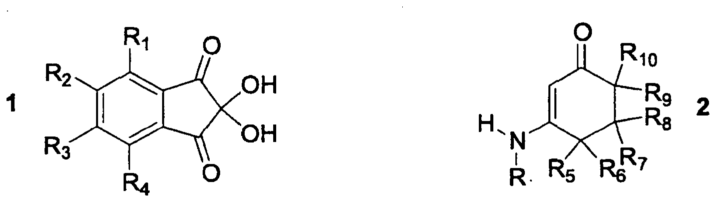
(2) Reaktion des Enaminons **2** mit Indantrionhydrat **1** zu der Additionsverbindung **3,** und 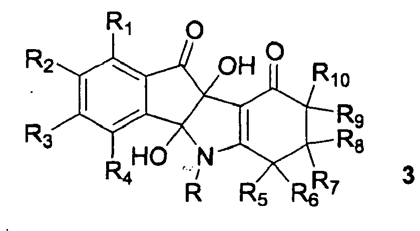
(3) Deoxygenierung der Verbindung 3, um die Verbindung **4** mit der Struktur I zu erhalten, 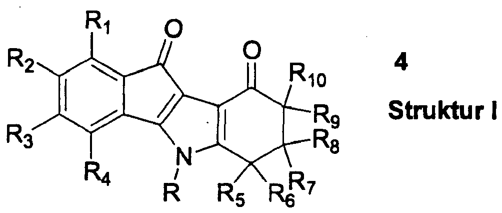 wobei R₅, R₆, R₈, und R₁₀ H sind und R, R₁, R₂, R₃, R₄, R₇, R₉ wie in den Ansprüchen 1 bis 20 definiert sind und X, Y, und Z Sauerstoff sind,
wobei Schritt (3) durchgeführt wird mit
(a) N,N,N',N'-Tetraalkylschwefligsäurediamid mit Alkyl = Methyl oder Ethyl oder
(b) Thionylchlorid und einer organischen basischen Substanz wie z.B. Natriumcarbonat oder einer organischen Base wie z.B. Trialkylamin R₃N, wobei R eine geradkettige oder verzweigte C₁-C₈-Alkylgruppe, insbesondere Methyl oder Ethyl, oder eine C₃-C₈-Cycloalkylgruppe ist, oder einem stickstoffhaltigen aromatischen Amin wie z.B. Imidazol oder Pyridin, oder
(c) Thionyldiimidazol,
oder zur Synthese einer Verbindung der Struktur II nach einem der Ansprüche 1 bis 10, umfassend die Schritte (1), (2) und (3) und die weiteren Schritte:
(4) Dehydrierung der Verbindungen **4,** was die Verbindung **5** ergibt, und. 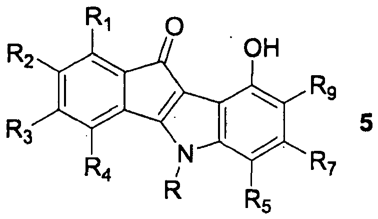
(5) Oxidation der Verbindung **5,** was Verbindung **6** mit Struktur II ergibt, 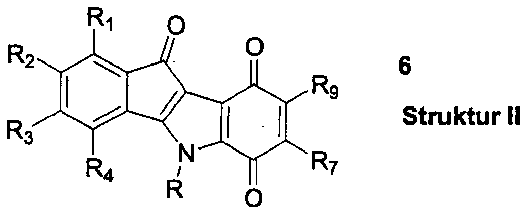 wobei R₅, R₆, R₈, und R₁₀ H sind und R, R₁, R₂, R₃, R₄, R₇, R₉ wie in den Ansprüchen 1 bis 10 definiert sind.

## Claims

1. Compound of structure I, structure IA or structure II 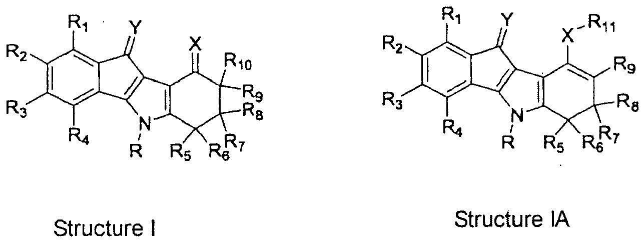 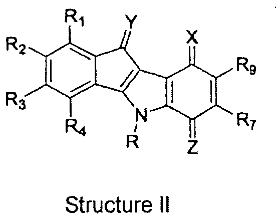 in which:
R represents
a hydrogen atom or
a straight-chain or branched C₁-C₈-alkyl group or
a C₃-C₈-cycloalkyl group or
a straight-chain or branched C₂-C₈-alkenyl group or
a C₃-C₈-cycloalkenyl group or
an aryl group or
a heteroaryl group or
an aryl-C₁-C₈-alkyl group or
a heteroaryl-C₁-C₈-alkyl group,
wherein R is optionally mono- or polysubstituted, either identically or differently, by hydroxy groups or
cyano groups or
nitro groups or
halogen atoms or
carboxy groups or
sulphonic acid groups or
groups of formula COOR₁₂ (alkoxycarbonyl group) or SO₂OR₁₃ (alkoxy sulphonic acid group), in which R₁₂ and R₁₃, which are identical or different, each represent a straight-chain or branched C₁-C₈-alkyl group or C₃-C₈-cycloalkyl group or a straight-chain or branched C₂-C₈-alkenyl group, optionally mono- or polysubstituted, either identically or differently, by hydroxy groups, cyano groups, nitro groups, halogen atoms, carboxy groups, or groups of the formulae NR₁₄R₁₅, CONR₁₄R₁₅, CSNR₁₄R₁₅, SO₂NR₁₄R₁₅, in which R₁₄ and R₁₅, which are identical or different, each represent a hydrogen atom or a straight-chain or branched C₁-C₈-alkyl group or a C₃-C₈-cycloalkyl group, or form, together with the nitrogen atom to which they are attached, a nitrogen-containing heterocycle, or
groups of the formulae CONR₁₆R₁₇ (carboxamide group), CSNR₁₆R₁₇ (thiocarboxamide group), SO₂NR₁₆R₁₇ (sulphonamide groups), in which R₁₆ and R₁₇, which are identical or different, each represent a hydrogen atom or a straight-chain or branched C₁-C₈-alkyl group or C₃-C₈-cycloalkyl group or a straight-chain or branched C₂-C₈-alkenyl group, or form, together with the nitrogen atom to which they are attached, a nitrogen-containing heterocycle, and are optionally mono- or polysubstituted, either identically or differently, by hydroxy groups, cyano groups, nitro groups, halogen atoms, carboxy groups, or
amino groups NR₁₈R₁₉, in which R₁₈ and R₁₉, which are identical or different, each represent a hydrogen atom or a straight-chain or branched C₁-C₈-alkyl group or C₃-C₈-cycloalkyl group or straight-chain or branched C₂-C₈-alkenyl group, or form, together with the nitrogen atom to which they are attached, a nitrogen-containing heterocycle, or
acyloxy groups OCOR₂₀, in which R₂₀ represents a straight-chain or branched C₁-C₈-alkyl group or C₃-C₈-cycloalkyl group or a straight-chain or branched C₂-C₈-alkenyl group, optionally identically or differently mono- or polysubstituted by hydroxy groups, cyano groups, nitro groups, halogen atoms, carboxy groups or groups of formulae COOR₂₁, NR₂₁R₂₂, CONR₂₁R₂₂, CSNR₂₁R₂₂, SO₂NR₂₁R₂₂, in which R₂₁ and R₂₂, which are identical or different, each represent a hydrogen atom or a straight-chain or branched C₁-C₈-alkyl group or a C₃-C₈-cycloalkyl group, or form, together with the nitrogen atom to which they are attached, a nitrogen-containing heterocycle, or
groups of formula OR₂₃NR₂₄R₂₅ (aminoalkyloxy), in which R₂₃ represents a straight-chain or branched C₁-C₈-alkyl group, and R₂₄ and R₂₅, which are identical or different, each represent a hydrogen atom or a straight-chain or branched C₁-C₈-alkyl group or a C₃-C₈-cycloalkyl group, or form, together with the nitrogen atom to which they are attached, a nitrogen-containing heterocycle,
wherein, independently of one another, R₁, R₂, R₃, R₄, R₅, R₆, R₇, R₈, R₉, R₁₀, which are identical or different, each represent
a hydrogen atom or
a straight-chain or branched C₁-C₈-alkyl group or
a C₃-C₈-cycloalkyl group or
a straight-chain or branched C₂-C₈-alkenyl group or
a C₃-C₈-cydoalkenyl group or
an aryl group or
a straight-chain or branched aryl-C₁-C₈-alkyl group or
a heteroaryl group or
a hydroxy group or
a straight-chain or branched C₁-C₈-alkoxy group or
a C₃-C₈-cydoalkoxy group or
a straight-chain or branched C₂-C₈-alkenyloxy group or
a C₃-C₈-cycloalkenyloxy group or
a C₁-C₂-alkylenedioxy group, which forms one of the groups R₁-R₁₀ with an adjacent group R₁-R₁₀, or
an acyloxy group OCOR₂₆, in which R₂₆ represents a straight-chain or branched C₁-C₈-alkyl group or a straight-chain or branched C₂-C₈-alkenyl group or a C₃-C₈-cycloalkyl group, or
a straight-chain or branched aryl-C₁-C₈-alkoxy group or
an alkoxycarbonylalkyl group R₂₇COOR₂₈, in which R₂₇ and R₂₈, which are identical or
different, are each a straight-chain or branched C₁-C₈-alkyl group or a C₃-C₈-cycloalkyl group or a straight-chain or branched C₂-C₈-alkenyl group, or
a carboxy group, COOH, or
a group of formulae CONR₂₉R₃₀ (carboxamide group), CSNR₂₉R₃₀ (thiocarboxamide group),
SO₂NR₂₉R₃₀ (sulphonamide group), in which R₂₉ and R₃₀, which are identical or different,
each represent a hydrogen atom or a straight-chain or branched C₁-C₈-alkyl group or C₃-C₈-cycloalkyl group, or form, together with the nitrogen atom to which they are attached, a nitrogen-containing heterocycle, or
an alkoxycarbonyl group COOR₃₁, in which R₃₁, is a straight-chain or branched C₁-C₈-alkyl group or a C₃-C₈-cycloalkyl group or a straight-chain or branched C₂-C₈-alkenyl group, or an amino group NR₃₂R₃₃, in which R₃₂ and R₃₃, which are identical or different, each represent a hydrogen atom or a straight-chain or branched C₁-C₈-alkyl group or C₃-C₈-cycloalkyl group, a straight-chain or branched C₂-C₈-alkenyl group, or form, together with the nitrogen atom to which they are attached, a nitrogen-containing heterocycle, or
an aminoalkyloxy group of formula OR₃₄NR₃₅R₃₆, in which R₃₄ represents a straight-chain or branched C₁-C₈-alkyl group or a C₃-C₈-cydoalkyl group or a straight-chain or branched C₂-C₈-alkenyl group, and R₃₅ and R₃₆, which are identical or different, each represent a hydrogen atom or a straight-chain or branched C₁-C₈-alkyl group or C₃-C₈-cycloalkyl group, or form, together with the nitrogen atom to which they are attached, a nitrogen-containing heterocycle,
wherein, independently of one another, each of the groups R₁, R₂, R₃, R₄, R₅, R₆, R₇, R₈, R₉, R₁₀ is optionally mono- or polysubstituted, either identically or differently, by hydroxy groups or
cyano groups or
nitro groups or
halogen atoms or
carboxy groups or
sulphonic acid groups or
aryl group or
heteroaryl group or
groups of formulae COOR₃₇, NR₃₇R₃₈, CONR₃₇R₃₈ (carboxamide group), CSNR₃₇R₃₈ (thiocarboxamide group), SO₂NR₃₇R₃₈ (sulphonamide group), in which R₃₇ and R₃₈, which are identical or different, each represent a hydrogen atom or a straight-chain or branched C₁-C₈-alkyl group or C₃-C₈-cycloalkyl group, or form, together with the nitrogen atom to which they are attached, a nitrogen-containing heterocycle, and are optionally mono- or polysubstituted, either identically or differently, by hydroxy groups, cyano groups, nitro groups, halogen atoms, carboxy groups or groups of formulae COOR₃₉, NR₃₉R₄₀, CONR₃₉R₄₀, CSNR₃₉R₄₀, SO₂NR₃₉R₄₀, in which R₃₉ and R₄₀, which are identical or different,
each represent a hydrogen atom or a straight-chain or branched C₁-C₈-alkyl group or a C₃-C₈-cycloalkyl group, or form, together with the nitrogen atom to which they are attached, a nitrogen-containing heterocycle,
wherein, independently of one another, X, Y, Z, which are identical or different, each represent
an oxygen atom,
an imino function NR₄₁, in which R₄₁ represents a hydrogen atom or a linear or branched C₁-C₈-alkyl group or a C₃-C₈-cycloalkyl group or a linear or branched C₂-C₈-alkenyl group or a C₃-C₈-cycloalkenyl group or an aryl group or heteroaryl group, or
an alkyloxyimino function NOR₄₂, in which R₄₂ represents a hydrogen atom or a linear or branched C₁-C₈-alkyl group or a C₃-C₈-cycloalkyl group or a linear or branched C₂-C₈-alkenyl group or a C₃-C₈-cycloalkenyl group, or an aryloxyimino function NOR₄₃, in which R₄₃ represents an aryl group or a heteroaryl group,
wherein, independently of one another, NR₄₁, NOR₄₂ and NOR₄₃ are optionally mono- or polysubstituted, either identically or differently, by aryl groups, heteroaryl groups, hydroxy groups, cyano groups, nitro groups, halogen atoms or by groups of formulae COOR₄₄, NR₄₄R₄₅ (amino group), CONR₄₄R₄₅ (carboxamide group), CSNR₄₄R₄₅ (thiocarboxamide group), SO₂NR₄₄R₄₅ (sulphonamide group), in which R₄₄ and R₄₅, which are identical or different, each represent a hydrogen atom or a straight-chain or branched C₁-C₈-alkyl group or a C₃-C₈-cycloalkyl group, or optionally form, together with the nitrogen atom to which they are attached, a nitrogen-containing heterocycle,
wherein X-R₁₁ in structure IA represents
a hydroxy group or
an alkoxy group, in which R₁₁ is a straight-chain or branched C₁-C₈-alkyl group or a C₃-C₈-cycloalkyl group or a straight-chain or branched C₂-C₈-alkylene group, optionally mono- or polysubstituted by aryl groups, heteroaryl groups, hydroxy groups, cyano groups, nitro groups, halogen atoms, carboxy groups or groups of formulae COOR₄₆, NR₄₆R₄₇, CONR₄₆R₄₇, CSNR₄₆R₄₇, SO₂NR₄₆R₄₇ in which R₄₆ and R₄₇, which are identical or different, each represent a hydrogen atom or a straight-chain or branched C₁-C₈-alkyl group or a C₃-C₈-cycloalkyl group, or form, together with the nitrogen atom to which they are attached, a nitrogen-containing heterocycle, or
an acyloxy group OCOR₄₈, in which R₄₈ represents a straight-chain or branched C₁-C₈-alkyl group or a straight-chain or branched C₂-C₈-alkenyl group or a C₃-C₈-cycloalkyl group or an aryl group or a heteroaryl group, optionally mono- or polysubstituted by aryl groups, heteroaryl groups, hydroxy groups, cyano groups, nitro groups, halogen atoms, carboxy groups or groups of formulae COOR₄₉, NR₄₉R₅₀, CONR₄₉R₅₀, CSNR₄₉R₅₀, SO₂NR₄₉R₅₀, in which R₄₉ and R₅₀, which may be identical or different, each represent a hydrogen atom or a straight-chain or branched C₁-C₈-alkyl group or a C₃-C₈-cycloalkyl group, or form, together with the nitrogen atom to which they are attached, a nitrogen-containing heterocycle,
wherein, independently of one another, C₂-C₈-alkenyl groups are each defined as straight-chain or branched hydrocarbon chains containing at least one double bond up to the maximum number of conjugated double bonds, and
wherein C₃-C₈-cycloalkenyl groups independently from each other are defined as cyclic hydrocarbons having at least one conjugated double bond up to the maximum number of conjugated double bonds, and
wherein, independently from one another, aryl groups are each defined as phenyl or naphthyl groups, which may be substituted with one or more identical or different substituents such as linear or branched C₁-C₈-alkyl groups, C₃-C₈-cycloalkyl groups, phenyl-C₁-C₈-alkyl group, naphthyl-C₁-C₈-alkyl group, C₁-C₂-alkylenedioxy group, hydroxy group, nitro group, cyano group, halogen atoms, linear or branched C₁-C₈-alkoxy groups, phenoxy group, linear or branched C₁-C₈-alkoxycarbonyl groups, carboxy group, groups of formulae OCR₅₁NR₅₂R₅₃ (amino-C₁-C₈-alkoxy group), NR₅₂R₅₃ (amino group), CONR₅₂R₅₃ (carboxamide group), CSNR₅₂R₅₃ (thiocarboxamide group), SO₂NR₅₂R₅₃ (sulphonamide group), in which R₅₁ represents a straight-chain or branched C₁-C₈-alkyl group, and R₅₂ and R₅₃, which are identical or different, each represent a hydrogen atom or a straight-chain or branched C₁-C₈-alkyl group or a C₃-C₈-cycloalkyl group, or form, together with the nitrogen atom to which they are attached forming a nitrogen-containing heterocycle,
wherein, independently of one another, heteroaryl groups are each defined as aromatic mono- or bicyclic 5 to 12 cyclic-membered ring systems which contain 1-3 hetero atoms (O, N, S) and may be substituted with one or more identical or different substituents such as linear or branched C₁-C₈-alkyl groups, C₃-C₈-cycloalkyl groups, linear or branched C₁-C₈-alkylene groups, aryl groups, hydroxy groups, nitro groups, cyano groups, halogen atoms, linear or branched C₁-C₈-alkoxy groups, C₃-C₈-cycloalkoxy groups, phenoxy groups, groups of formulae OCR₅₄NR₅₅R₅₆ (amino-C₁-C₈-alkoxy group), NR₅₅R₅₆ (amino group), CONR₅₅R₅₆ (carboxamide group), CSNR₅₅R₅₆ (thiocarboxamide group), SO₂NR₅₅R₅₆ (sulphonamide group), in which R₅₄ represents a straight-chain or branched C₁-C₈-alkyl group, and R₅₅ and R₅₆, which may be identical or different, each represent a hydrogen atom or a straight-chain or branched C₁-C₈-alkyl group or a C₃-C₈-cycloalkyl group, or form, together with the nitrogen atom to which they are attached, a nitrogen-containing heterocycle, and
wherein, independently of one another, a nitrogen-containing heterocycle is defined in each case as a saturated 5 to 7 cyclic-membered monocycle which, in addition to nitrogen, contains 1-3 heteroatoms (preferably O, N, S), in particular pyrrolidinyl-, piperidyl-, morpholinyl- and piperazinyl groups,
or a salt thereof with a pharmaceutically acceptable acid, wherein a pharmaceutically acceptable acid is defined as in particular hydrochloric acid, hydrobromic acid, sulphuric acid, phosphoric acid, acetic acid, trifluoroacetic acid, lactic acid, pyruvic acid, malonic acid, succinic acid, glutaric acid, tartaric acid, citric acid, ascorbic acid, oxalic acid,
or a mixture of isomers or a pure isomer thereof, wherein isomers are optical isomers and geometric isomers with regard to double bonds, in particular diastereomers, enantiomer and E/Z isomers.

2. Compound according to claim 1, in which R represents
a hydrogen atom or
a straight-chain or branched C₁-C₈-alkyl group or
a C₃-C₈-cycloalkyl group or
a straight-chain or branched C₂-C₈-alkenyl group or
a C₃-C₈-cycloalkenyl group or
an aryl group or
a heteroaryl group, or
an amino group NR₃₂R₃₃, or
a straight-chain or branched C₁-C₈-alkoxy group or
a C₃-C₈-cycloalkoxy group,
which are as defined in claim 1 and optionally substituted as defined in claim 1.

3. Compound according to either claim 1 or claim 2, wherein, independently of one another, R₁, R₂, R₃, R₄, R₅, R₆, R₇, R₈, R₉, R₁₀, which are identical or different, each represent
a hydrogen atom or
a straight-chain or branched C₁-C₈-alkyl group or
a C₃-C₈-cycloalkyl group or
a straight-chain or branched C₂-C₈-alkenyl group or
a C₃-C₈-cycloalkenyl group or
an aryl group or
a heteroaryl group or
a hydroxy group or
an acyloxy group OCOR₂₆,
a carboxy group COOH, or
an amino group NR₃₂R₃₃, or
a straight-chain or branched C₁-C₈-alkoxy group or
a C₁-C₂-alkylenedioxy group,
which are as defined in claim 1 and optionally substituted as defined in claim 1.

4. Compound according to any of claims 1 to 3, in which X is an oxygen atom and/or
Y is an oxygen atom and/or
Z is an oxygen atom.

5. Compound according to any of claims 1 to 3, in which R₅-R₁₀ are hydrogen, and/or R₇ and R₉ are hydrogen.

6. Compound according to any of claims 1 to 3, in which R₁-R₁₀ are identical or different and are hydrogen, straight-chain or branched C₁-C₈-alkyl groups or straight-chain or branched C₁-C₈-alkoxy groups.

7. Compound according to any of claims 1 to 3, in which R is a hydrogen atom or a linear or branched C₁-C₈-alkyl group or an aryl group as defined in claim 1, or is a linear or branched aryl-C₁-C₈-alkyl group as defined in claim 1.

8. Compound according to any of claims 1 to 3 having the structure I having the IUPAC name
*5-isopropyl-5,6,7,8-tetrahydro-indeno[1,2-b]indole-9,10-dione,*
*5-phenethyl-5,6,7,8-tetrahydro-indeno[1,2-b]indole-9,10-dione,*
*5-(1-phenyl-ethyl)-5,6,7,8-tetrahydro-indeno[1,2-b]indole-9,10-dione,*
its isomers or
*5-phenyl-5,6,7,8-tetrahydro-indeno[1,2-b]indol-9,10-dione,*
or a salt thereof having a pharmaceutically acceptable acid.

9. Compound according to any of claims 1 to 3 having the structure II having the IUPAC name
*5-isopropyl-5H-indeno[1,2-b]indole-6,9,10-trione,*
*5-benzyl-5,6,9,10-tetrahydroindeno[1,2-b]indole-6,9,10-trione,* or
*5-phenethyl-5H-indeno[1,2-b]indole-6,9,10-trione,*
or a salt thereof having a pharmaceutically acceptable acid.

10. Compound according to any of claims 1 to 3 having the structure IA, wherein XR₁₁ is OR₁₁, and wherein R₁₁ is as defined in claim 1.

11. Use of a compound according to any of claims 1 to 10 for the *in vitro* inhibition of a protein kinase,
wherein the compound according to any of claims 1 to 10 is optionally used in combination with a further protein kinase inhibiting agent.

12. Use of a compound according to any of claims 1 to 10 for producing a drug for treating cancer and/or a tumour or for producing a diagnostic agent for cancer and/or a tumour disease.

13. Use of compound according to any of the claims 1 to 10 for producing a drug for treating an inflammatory disease or for producing a diagnostic agent for an inflammatory disease.

14. Pharmaceutical preparation,
**characterised in that** it contains at least one compound of structure I, structure IA or structure II according to any of claims 1 to 10 as a substance in an effective amount and was optionally prepared with at least one pharmaceutically acceptable additive or/and adjuvant, optionally comprising at least one further substance.

15. Method for the synthesis of a compound of the structure I according to any of claims 1 to 10, comprising the steps of:
(1) preparing a cyclic enaminone 2 from a cyclic 1,3-diketone by reaction with ammonia or/and a primary amine and provision of an indanetrione 1, 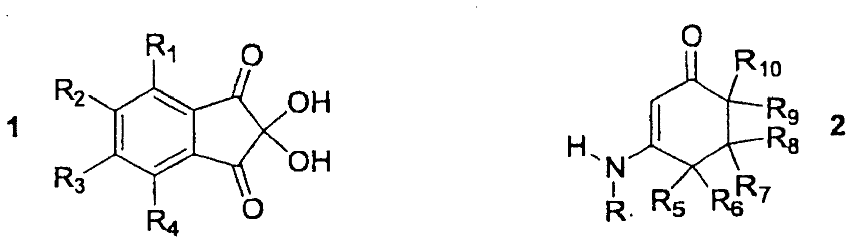
(2) reacting the enaminone 2 with indanetrione hydrate 1 to the addition compound 3, and 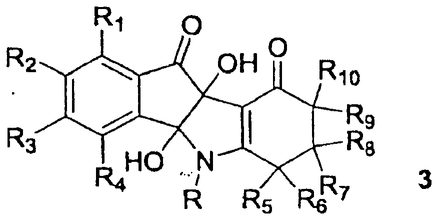
(3) deoxygenating the compound 3 to obtain the compound 4 having structure I, 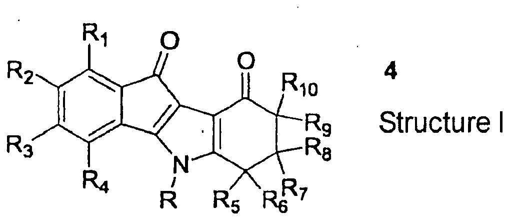 wherein R₅, R₆, R₈ and R₁₀ are H and R, R₁, R₂, R₃, R₄, R₇ and R₉ are as defined in claims 1 to 10 and X, Y and Z are oxygen
wherein step (3) is carried out with
(a) N,N,N',N'-tetralkyl sulphurous acid diamide with alkyl=methyl or ethyl or
(b) thionylchloride and an organic alkaline substance such as sodium carbonate or an organic base such as trialkyl amine R₃N, wherein R is a straight-chain or branched C₁-C₈-alkyl group, in particular methyl or ethyl, or a C₃-C₈-cycloalkyl group, or a nitrogen-containing aromatic amine such as imidazole or pryridine, or
(c) thionyldiimidazole,
or for the synthesis of a compound of structure II according to any of claims 1 to 10, comprising steps (1), (2) and (3) and the further steps of:
(4) dehydrogenating the compounds 4, resulting in the compound 5, and 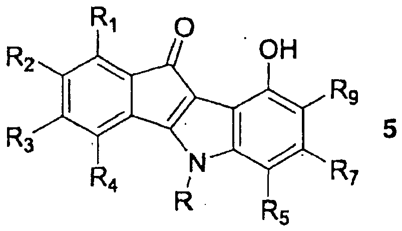
(5) oxidising the compound 5, resulting in the compound 6 having structure II, 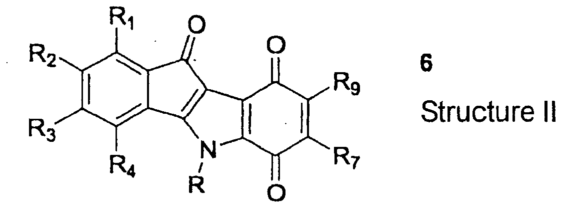 wherein R₅, R₆, R₈ and R₁₀ are H and R, R₁, R₂, R₃, R₄, R₇ and R₉ are as defined in claims 1 to 10.

## Revendications

1. Composé de la structure I, la structure IA ou la structure II 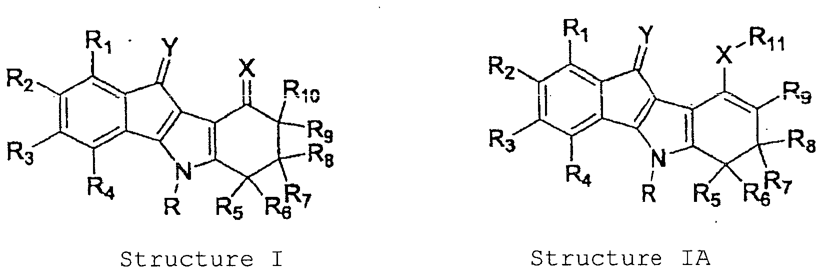 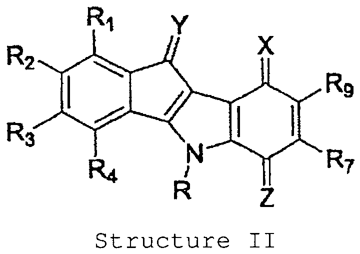 dans lesquelles :
R signifie l'atome d'hydrogène, ou un radical alkyle en C₁-C₈ linéaire ou ramifié, ou un radical cycloalkyle en C₃-C₈, ou un radical alcényle en C₂-C₈ linéaire ou ramifié, ou un radical cycloalcényle en C₃-C₈, ou un radical aryle, ou un radical hétéroaryle, un radical aryl(alkyle en C₁-C₈) ou un radical hétéroaryl(alkyle en C₁-C₈)
où R est substitué le cas échéant, une ou plusieurs fois, de manière identique ou différente par des radicaux hydroxyle, ou des radicaux cyano, ou des radicaux nitro, ou des atomes d'halogène, ou des radicaux carboxy, ou des radicaux acide sulfonique ou
des radicaux de formule COOR₁₂ (radical alcoxycarbonyle) ou SO₂OR₁₃ (radical acide alcoxysulfonique), où R₁₂, R₁₃, qui sont identiques ou différents, représentent chacun un radical alkyle en C₁-C₈ linéaire ou ramifié, ou un radical cycloalkyle en C₃-C₈, ou un radical alcényle en C₂-C₈ linéaire ou ramifié, qui sont substitués le cas échéant, une ou plusieurs fois, de manière identique ou différente par des radicaux hydroxyle, des radicaux cyano, des radicaux nitro, des atomes d'halogène, des radicaux carboxy, ou des radicaux des formules NR₁₄R₁₅, CONR₁₄R₁₅, CSNR₁₄R₁₅, SO₂NR₁₄R₁₅, où R₁₄, R₁₅, qui sont identiques ou différents, représentent chacun l'atome d'hydrogène, ou un radical alkyle en C₁-C₈ linéaire ou ramifié ou un radical cycloalkyle en C₃-C₈, ou ensemble, ils forment un hétérocycle azoté avec l'atome d'azote qui les porte, ou
des radicaux de formules CONR₁₆R₁₇ (radical carboxamido), CSNR₁₆R₁₇ (radical thiocarboxamido), SO₂NR₁₆R₁₇ (radical sulfonamido), où R₁₆, R₁₇, qui sont identiques ou différents, représentent chacun l'atome d'hydrogène, ou un radical alkyle en C₁-C₈ linéaire ou ramifié, ou un radical cycloalkyle en C₃-C₈, ou un radical alcényle en C₂-C₈ linéaire ou ramifié, ou ensemble, ils forment un hétérocycle azoté avec l'atome d'azote qui les porte, et qui sont substitués le cas échéant, une ou plusieurs fois, de manière identique ou différente par des radicaux hydroxyle, des radicaux cyano, des radicaux nitro, des atomes d'halogène, des radicaux carboxy, ou
des radicaux amino NR₁₈R₁₉, où R₁₈, R₁₉, qui sont identiques ou différents, représentent chacun l'atome d'hydrogène, ou un radical alkyle en C₁-C₈ linéaire ou ramifié, un radical cycloalkyle en C₃-C₈, ou un radical alcényle en C₂-C₈ linéaire ou ramifié, ou ensemble, ils forment un hétérocycle azoté avec l'atome d'azote qui les porte, ou
des radicaux acyloxy OCOR₂₀, dans lesquels R₂₀ représente un radical alkyle en C₁-C₈ linéaire ou ramifié, ou un radical cycloalkyle en C₃-C₈, ou un radical alcényle en C₂-C₈ linéaire ou ramifié, qui est substitué le cas échéant, une ou plusieurs fois, de manière identique ou différente par des radicaux hydroxyle, des radicaux cyano, des radicaux nitro, des atomes d'halogène, des radicaux carboxy, ou des radicaux de formules COOR₂₁, NR₂₁R₂₂, CONR₂₁R₂₂, CSNR₂₁R₂₂, SO₂NR₂₁R₂₂, où R₂₁, R₂₂, qui sont identiques ou différents, représentent chacun l'atome d'hydrogène, ou un radical alkyle en C₁-C₈ linéaire ou ramifié ou un radical cycloalkyle en C₃-C₈, ou ensemble, ils forment un hétérocycle azoté avec l'atome d'azote qui les porte, ou
des groupes de formule OR₂₃NR₂₄R₂₅ (aminoalcoxy), où R₂₃ représente un radical alkyle en C₁-C₈ linéaire ou ramifié, R₂₄, R₂₅, qui sont identiques ou différents, représentent chacun l'atome d'hydrogène, ou un radical alkyle en C₁-C₈ linéaire ou ramifié, ou un radical cycloalkyle en C₃-C₈, ou ensemble, ils forment un hétérocycle azoté avec l'atome d'azote qui les porte,
où R₁, R₂, R₃, R₄, R₅, R₆, R₇, R₈, R₉, R₁₀, qui sont identiques ou différents, représentent chaque fois indépendamment l'un de l'autre
l'atome d'hydrogène, ou un radical alkyle en C₁-C₈ linéaire ou ramifié, ou un radical cycloalkyle en C₃-C₈, ou un radical alcényle en C₂-C₈ linéaire ou ramifié, ou un radical cycloalcényle en C₃-C₈, ou un radical aryle, ou un radical aryl(alkyle en C₁-C₈) linéaire ou ramifié, ou un radical hétéroaryle, ou le radical hydroxyle, ou un radical alcoxy en C₁-C₈ linéaire ou ramifié, ou un radical cycloalcoxy en C₃-C₈, ou un radical alcényloxy en C₂-C₈ linéaire ou ramifié, ou un radical cycloalcényloxy en C₃-C₈, ou un radical alkylènedioxy en C₁-C₂, qui est formé avec un des radicaux R₁-R₁₀ avec le radical R₁-R₁₀ voisin, ou
un radical acyloxy OCOR₂₆, où R₂₆ représente un radical alkyle en C₁-C₈ linéaire ou ramifié, ou un radical alcényle en C₂-C₈ linéaire ou ramifié, ou un radical cycloalkyle en C₃-C₈, ou
un radical aryl(alcoxy en C₁-C₈) linéaire ou ramifié, ou
un radical alcoxycarbonylalkyle R₂₇COOR₂₈, où R₂₇, R₂₈, qui sont identiques ou différents, représentent chacun un radical alkyle en C₁-C₈ linéaire ou ramifié, ou un radical cycloalkyle en C₃-C₈, ou un radical alcényle en C₂-C₈ linéaire ou ramifié, ou
le radical carboxy COOH, ou
un radical de formule CONR₂₉R₃₀ (radical carboxamido), CSNR₂₉R₃₀ (radical thiocarboxamido), SO₂NR₂₉R₃₀ (radical sulfonamido), où R₂₉, R₃₀, qui sont identiques ou différents, représentent chacun l'atome d'hydrogène, ou un radical alkyle en C₁-C₈ linéaire ou ramifié ou un radical cycloalkyle en C₃-C₈, ou ensemble, ils forment un hétérocycle azoté avec l'atome d'azote qui les porte, ou
un radical alcoxycarbonyle COOR₃₁, où R₃₁ est un radical alkyle en C₁-C₈ linéaire ou ramifié, un radical cycloalkyle en C₃-C₈, ou un radical alcényle en C₂-C₈, linéaire ou ramifié, ou
un radical amino NR₃₂R₃₃, où R₃₂, R₃₃, qui sont identiques ou différents, représentent chacun l'atome d'hydrogène, ou un radical alkyle en C₁-C₈ linéaire ou ramifié, ou un radical cycloalkyle en C₃-C₈, ou un radical alcényle en C₂-C₈ linéaire ou ramifié, ou ensemble, ils forment un hétérocycle azoté avec l'atome d'azote qui les porte, ou
un radical aminoalcoxy de formule OR₃₄NR₃₅R₃₆, où R₃₄ représente un radical alkyle en C₁-C₈ linéaire ou ramifié, ou un radical cycloalkyle en C₃-C₈ ou un radical alcényle en C₂-C₈ linéaire ou ramifié, R₃₅, R₃₆, qui sont identiques ou différents, représentent chacun l'atome d'hydrogène, ou un radical alkyle en C₁-C₈ linéaire ou ramifié, ou un radical cycloalkyle en C₃-C₈, ou ensemble, ils forment un hétérocycle azoté avec l'atome d'azote qui les porte,
où chacun des radicaux R₁, R₂, R₃, R₄, R₅, R₆, R₇, R₈, R₉, R₁₀ est le cas échéant substitué, une ou plusieurs fois, de manière identique ou différente et indépendamment l'un de l'autre, par des radicaux hydroxyle, ou des radicaux cyano, ou des radicaux nitro, ou des atomes d'halogène, ou des radicaux carboxy, ou des radicaux acide sulfonique, ou des radicaux aryle, ou des radicaux hétéroaryle, des radicaux des formules COOR₃₇, NR₃₇R₃₈, CONR₃₇R₃₈ (radical carboxamido), CSNR₃₇R₃₈ (radical thiocarboxamido), SO₂NR₃₇R₃₈ (radical sulfonamido), où R₃₇, R₃₈, qui sont identiques ou différents, représentent l'atome d'hydrogène, ou un radical alkyle en C₁-C₈ linéaire ou ramifié ou un radical cycloalkyle en C₃-C₈, ou ensemble, ils forment un hétérocycle azoté avec l'atome d'azote qui les porte, et qui sont substitués le cas échéant, une ou plusieurs fois, de manière identique ou différente par des radicaux hydroxyle, des radicaux cyano, des radicaux nitro, des atomes d'halogène, des radicaux carboxy, ou des radicaux des formules COOR₃₉, NR₃₉R₄₀, CONR₃₉R₄₀, CSNR₃₉R₄₀, SO₂NR₃₉R₄₀, où R₃₉, R₄₀, qui sont identiques ou différents, représentent chacun l'atome d'hydrogène, un radical alkyle en C₁-C₈ linéaire ou ramifié, ou un radical cycloalkyle en C₃-C₈, ou ensemble, ils forment un hétérocycle azoté avec l'atome d'azote qui les porte,
où X, Y, Z, qui sont identiques ou différents, représentent indépendamment l'un de l'autre,
l'atome d'oxygène,
une fonction imino NR₄₁, où R₄₁ représente l'atome d'hydrogène, ou un radical alkyle en C₁-C₈ linéaire ou ramifié, ou un radical cycloalkyle en C₃-C₈, ou un radical alcényle en C₂-C₈ linéaire ou ramifié, ou un radical cycloalcényle en C₃-C₈, ou un radical aryle ou un radical hétéroaryle, ou
une fonction alkyloximino, NOR₄₂, où R₄₂ représente l'atome d'hydrogène, ou un radical alkyle en C₁-C₈ linéaire ou ramifié, ou un radical cycloalkyle en C₃-C₈, ou un radical alcényle en C₂-C₈ linéaire ou ramifié, ou un radical cycloalcényle en C₃-C₈, ou
une fonction aryloximino NOR₄₃, où R₄₃ représente un radical aryle ou un radical hétéroaryle,
où NR₄₁, NOR₄₂ et NOR₄₃ sont, le cas échéant, une ou plusieurs fois, substitués de manière identique ou différente par des radicaux aryle, des radicaux hétéroaryle, des radicaux hydroxy, des radicaux cyano, des radicaux nitro, des atomes d'halogène, ou des radicaux de formules COOR₄₄, NR₄₄R₄₅ (radical amino), CONR₄₄R₄₅ (radical carboxamido), CSNR₄₄R₄₅ (radical thiocarboxamido), SO₂NR₄₄R₄₅ (radical sulfonamide), où R₄₄, R₄₅, qui sont identiques ou différents, représentent chacun l'atome d'hydrogène, ou un radical alkyle en C₁-C₈ linéaire ou ramifié ou un radical cycloalkyle en C₃-C₈, ou ensemble, ils forment un hétérocycle azoté avec l'atome d'azote qui les porte,
où X-R₁₁ dans la structure IA représente un radical hydroxy, ou
un radical alcoxy, dans lequel R₁₁ est un radical alkyle en C₁-C₈ linéaire ou ramifié, ou un radical cycloalkyle en C₃-C₈, ou un radical alcényle en C₂-C₈ linéaire ou ramifié, qui est substitué le cas échéant, une ou plusieurs fois, par des radicaux aryle, des radicaux hétéroaryle, des radicaux hydroxyle, des radicaux cyano, des radicaux nitro, des atomes d'halogène, des radicaux carboxy, ou des radicaux de formules COOR₄₆, NR₄₆R₄₇, CONR₄₆R₄₇, CSNR₄₆R₄₇, SO₂NR₄₆R₄₇, où R₄₆, R₄₇, qui sont identiques ou différents, représentent chacun l'atome d'hydrogène, ou un radical alkyle en C₁-C₈ linéaire ou ramifié ou un radical cycloalkyle en C₃-C₈, ou ensemble, ils forment un hétérocycle azoté avec l'atome d'azote qui les porte, ou
un radical alcyloxy OCOR₄₈, dans lequel R₄₈ est un radical alkyle en C₁-C₈ linéaire ou ramifié, ou un radical alcényle en C₂-C₈ linéaire ou ramifié, ou un radical cycloalkyle en C₃-C₈, qui est substitué le cas échéant, une ou plusieurs fois, par des radicaux aryle, des radicaux hétéroaryle, des radicaux hydroxyle, des radicaux cyano, des radicaux nitro, des atomes d'halogène, des radicaux carboxy, ou des radicaux de formules COOR₄₉, NR₄₉R₅₀, CONR₄₉R₅₀, CSNR₄₉R₅₀, SO₂NR₄₉R₅₀, où R₄₉, R₅₀, qui peuvent être identiques ou différents, représentent chacun l'atome d'hydrogène ou un radical alkyle en C₁-C₈ linéaire ou ramifié ou un radical cycloalkyle en C₃-C₈, ou ensemble, ils forment un hétérocycle azoté avec l'atome d'azote qui les porte,
où par radical alcényle en C₂-C₈, on entend chaque fois indépendamment l'un de l'autre, une chaine hydrocarbure linéaire ou ramifiée, qui contient au moins jusqu'au nombre maximal de liaisons doubles conjuguées, et
où par radical cycloalcényle en C₃-C₈, on entend chaque fois indépendamment l'un de l'autre, un hydrocarbure cyclique qui contient au moins jusqu'au nombre maximal de liaisons doubles conjuguées, et
où par radical aryle, on entend chaque fois indépendamment l'un de l'autre, des radicaux phényle ou naphtyle, qui peuvent être substitués par un ou plusieurs substituants identiques ou différents, comme des radicaux alkyle en C₁-C₈ linéaires ou ramifiés, des radicaux cycloalkyle en C₃-C₈, un radical phényl(alkyle en C₁-C₈), un radical naphtyl(alkyle en C₁-C₈), un radical alkylènedioxy en C₁-C₂, le radical hydroxy, le radical nitro, le radical cyano, un atome d'halogène, des radicaux alcoxy en C₁-C₈ linéaires ou ramifiés, un radical phénoxy, des radicaux alcoxycarbonyle en C₁-C₈ linéaires ou ramifiés, le radical carboxy, des radicaux de formules OCR₅₁NR₅₂R₅₃ (aminoalcoxy en C₁-C₈), NR₅₂R₅₃ (radical amino), CONR₅₂R₅₃ (radical carboxamido), CSNR₅₂R₅₃ (radical thiocarboxamido), SO₂NR₅₂R₅₃ (radical sulfonamido), où R₅₁ représente un radical alkyle en C₁-C₈ linéaire ou ramifié, R₅₂, R₅₃, qui sont identiques ou différents, représentent chacun l'atome d'hydrogène, ou un radical alkyle en C₁-C₈ linéaire ou ramifié, ou un radical cycloalkyle en C₃-C₈, ou ensemble, ils forment un hétérocycle azoté avec l'atome d'azote qui les porte,
où par radical hétéroaryle, on entend chaque fois indépendamment l'un de l'autre, des systèmes aromatique mono- ou bicyclique de 5 à 12 membres, qui contiennent 1 à 3 hétéroatomes (O, N, S), qui peuvent être substitués par un ou plusieurs substituants identiques ou différents, comme des radicaux alkyle en C₁-C₈ linéaires ou ramifiés, des radicaux cycloalkyle en C₃-C₈, des radicaux alkylène en C₁-C₈ linéaires ou ramifiés, des radicaux aryle, le radical hydroxy, des radicaux nitro, des radicaux cyano, un atome d'halogène, des radicaux alcoxy en C₁-C₈ linéaires ou ramifiés, des radicaux cycloalcoxy en C₃-C₈, un radical phénoxy, des radicaux de formules OCR₅₄NR₅₅R₅₆ (aminoalcoxy en C₁-C₈), NR₅₅R₅₆ (radical amino), CONR₅₅R₅₆ (radical carboxamido), CSNR₅₅R₅₆ (radical thiocarboxamido), SO₂NR₅₅R₅₆ (radical sulfonamido), où R₅₄ représente un radical alkyle en C₁-C₈ linéaire ou ramifié, R₅₅, R₅₆, qui peuvent être identiques ou différents, représentent chacun l'atome d'hydrogène, ou un radical alkyle en C₁-C₈ linéaire ou ramifié, ou un radical cycloalkyle en C₃-C₈, ou ensemble, ils forment un hétérocycle azoté avec l'atome d'azote qui les porte, et
où par hétérocycle azoté, on entend chaque fois indépendamment l'un de l'autre, un monocycle saturé de 5 à 7 membres, qui contient en plus de l'azote, 1 à 3 hétéroatomes (de préférence, O, N, S), en particulier les radicaux pyrrolidinyle, pipéridyle, morpholinyle et pipérazinyle,
ou un sel de ceux-ci avec un acide pharmaceutiquement utilisable, où par acide pharmaceutiquement utilisable, on entend en particulier l'acide chlorhydrique, l'acide bromhydrique, l'acide sulfurique, l'acide phosphorique, l'acide acétique, l'acide trifluoroacétique, l'acide lactique, l'acide pyruvique, l'acide malonique, l'acide succinique, l'acide glutarique, l'acide tartrique, l'acide citrique, l'acide ascorbique, l'acide oxalique,
ou un mélange d'isomères ou un isomère pur de ceux-ci, où par isomère, on entend les isomères optiques et les isomères géométriques sur les doubles liaisons, en particulier les diastéréoisomères, les énantiomères et les isomères E/Z.

2. Composé selon la revendication 1, où R signifie l'atome d'hydrogène, ou
un radical alkyle en C₁-C₈ linéaire ou ramifié, ou un radical cycloalkyle en C₃-C₈, ou
un radical alcényle en C₂-C₈ linéaire ou ramifié, ou
un radical cycloalcényle en C₃-C₈, ou
un radical aryle, ou
un radical hétéroaryle, ou
un radical amino NR₃₂R₃₃, ou
un radical alcoxy en C₁-C₈ linéaire ou ramifié, ou
un radical cycloalcoxy en C₃-C₈,
qui sont tels que défini à la revendication 1 et le cas échéant, substitués comme défini à la revendication 1.

3. Composé selon la revendication 1 ou 2, où R₁, R₂, R₃, R₄, R₅, R₆, R₇, R₈, R₉, R₁₀, qui sont identiques ou différents, représentent chaque fois indépendamment l'un de l'autre
l'atome d'hydrogène, ou
un radical alkyle en C₁-C₈ linéaire ou ramifié,
un radical cycloalkyle en C₃-C₈, ou
un radical alcényle en C₂-C₈ linéaire ou ramifié,
un radical cycloalcényle en C₃-C₈, ou
un radical aryle, ou
un radical hétéroaryle, ou
le radical hydroxy, ou
un radical acyloxt OCOR₂₆, ou
le radical carboxy COOH, ou
un radical amino NR₃₂R₃₃, ou
un radical alcoxy en C₁-C₈ linéaire ou ramifié, ou
un radical alkylènedioxy en C₁-C₂,
qui sont tels que défini à la revendication 1 et le cas échéant, substitués comme défini à la revendication 1.

4. Composé selon l'une des revendications 1 à 3, où X est l'atome d'oxygène, et/ou
Y est l'atome d'oxygène, et/ou
Z est l'atome d'oxygène.

5. Composé selon l'une des revendications 1 à 3, où R₅-R₁₀ sont l'hydrogène, et/ou
R₇ et R₉ sont l'hydrogène.

6. Composé selon l'une des revendications 1 à 3, où R₁-R₁₀ sont identiques ou différents et sont hydrogène, un radical alkyle en C₁-C₈ linéaire ou ramifié ou un radical alcoxy en C₁-C₈ linéaire ou ramifié.

7. Composé selon l'une des revendications 1 à 3, où R est hydrogène, un radical alkyle en C₁-C₈ linéaire ou ramifié, ou un radical aryle comme défini à la revendication 1 ou un radical aryl(alkyle en C₁-C₈) linéaire ou ramifié comme défini à la revendication 1.

8. Composé selon l'une des revendications 1 à 3, ayant la structure I avec la dénomination IUPAC
5-isopropyl-5,6,7,8-tétrahydroindéno[1,2-b]indol-9,10-dione,
5-phénéthyl-5,6,7,8-tétrahydroindéno[1,2-b]indol-9,10-dione,
5-(1-phényléthyl)-5,6,7,8-tétrahydroindéno[1,2-b]indol-9,10-dione,
leurs isomères, ou
5-phényl-5,6,7,8-tétrahydroindéno[1,2-b]indol-9,10-dione,
ou un sel de ceux-ci, avec un acide pharmaceutiquement utilisable.

9. Composé selon l'une des revendications 1 à 3, ayant la structure II avec la dénomination IUPAC
5-isopropyl-5H-indéno[1,2-b]indol-6,9,10-trione,
5-benzyl-5,6,9,10-tétrahydroindéno[1,2-b]indol-6,9,10-trione,
5-phénéthyl-5H-indéno[1,2-b]indol-6,9,10-trione,
ou un sel de ceux-ci, avec un acide pharmaceutiquement utilisable.

10. Composé selon l'une des revendications 1 à 3 avec la structure IA, où XR₁₁ est OR₁₁ et où R₁₁ est comme défini à la revendication 1.

11. Utilisation d'un composé selon l'une des revendications 1 à 10, pour inhiber une protéine kinase *in vitro*,
où le composé selon l'une des revendications 1 à 10 est mis en oeuvre le cas échéant, en combinaison avec un autre inhibiteur de protéine kinase.

12. Utilisation d'un composé selon l'une des revendications 1 à 10, pour la préparation d'un médicament pour le traitement de cancers ou/et d'une tumeur, ou pour la préparation d'un produit de diagnostic pour les cancers et/ou une maladie tumorale.

13. Utilisation d'un composé selon l'une des revendications 1 à 10, pour la préparation d'un médicament pour le traitement d'une maladie inflammatoire, ou pour la préparation d'un diagnostic pour une maladie inflammatoire.

14. Composition pharmaceutique, **caractérisée en ce qu'**elle contient comme agent actif, au moins un composé de structure I, de structure IA ou de structure II selon l'une des revendications 1 à 10, en un dosage actif, qu'elle est préparée le cas échéant, avec au moins un additif et/ou auxiliaire utilisable pharmaceutiquement, et qu'elle comprend le cas échéant, au moins un autre agent actif.

15. Procédé pour la synthèse d'un composé de structure I selon l'une des revendications 1 à 10, comprenant les étapes de :
(1) Préparation d'une ènaminone cyclique 2 à partir d'une 1,3-dicétone cyclique, par réaction avec l'ammoniac et/ou une amine primaire, et préparation d'une indanetrione 1, 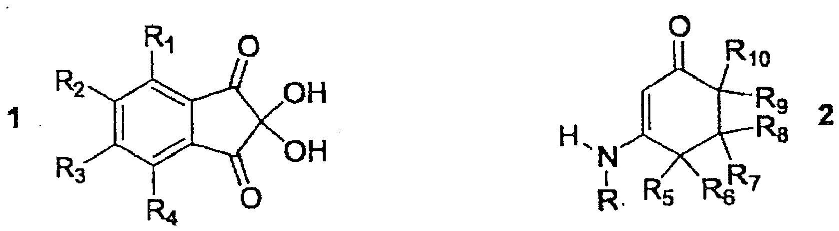
(2) Réaction de l'ènaminone 2 avec l'hydrate d'indanetrione 1 en le composé d'addition 3, et 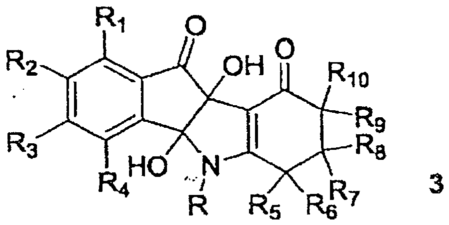
(3) Désoxygénation du composé 3, pour obtenir le composé 4 avec la structure I 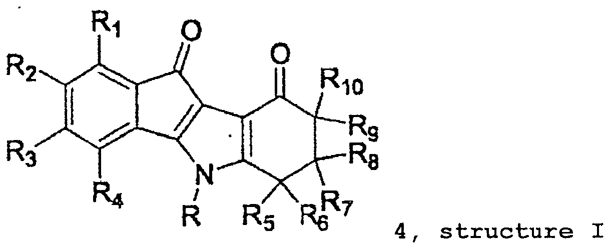 où R₅, R₆, R₈, et R₁₀ sont H et R, R₁, R₂, R₃, R₄, R₇, R₉ sont comme défini aux revendications 1 à 10, et X, Y et Z sont oxygène,
où l'étape (3) est réalisée avec
(a) un N,N,N',N'tétraalkyldiamide d'acide sulfureux avec aklyle = méthyle ou éthyle, ou
(b) le chlorure de thionyle et une substance inorganique basique, comme par exemple le carbonate de sodium, ou une base organique, comme par exemple une trialkylamine R₃N, où R est un radical alkyle en C₁-C₈ linéaire ou ramifié, en particulier éthyle ou méthyle, ou un radical cycloalkyle en C₃-C₈, ou une amine aromatique comme par exemple l'imidazole ou la pyridine, ou
(c) le thionyldiimidazole,
ou pour la synthèse d'un composé de structure II selon l'une des revendications 1 à 10, comprenant les étapes (1), (2) et (3) et les étapes supplémentaires de :
(4) Déshydratation du composé 4, ce qui donne le composé 5, et 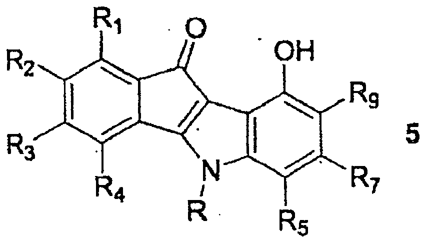
(5) Oxydation du composé 5, ce qui donne le composé 6 de structure II 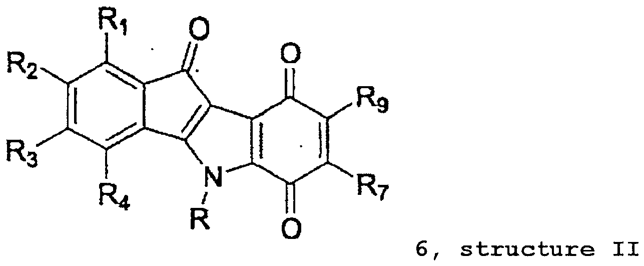 où R₅, R₆, R₈, et R₁₀ sont H et R, R₁, R₂, R₃, R₄, R₇, R₉ sont comme défini aux revendications 1 à 10.
